# EUROPEAN PATENT APPLICATION

(11) **EP 4 814 006 A2**
(43) Date of publication of application: **30.09.2026**
(21) Application number: 26198537.8
(22) Date of filing: 23.05.2025
(51) Int. Cl.: C07K 14/71

(54) **NOVEL BINDING PROTEINS FOR HYALURONIC ACID**

(30) Priority: 24.05.2024 EP 24178035; 27.06.2024 EP 24185152; 30.09.2024 EP 24203746
(62) Document number(s) of the earlier application(s) in accordance with Art. 76 EPC: 25728127.9 / 4 770 660
(71) Applicant: NAVIGO PROTEINS GMBH, 06120 Halle/Saale (DE)
(72) Inventor: HAUPTS, Ulrich, 06120 Halle/Saale (DE); COBURGER, Ina, 06120 Halle/Saale (DE); HOFFMANN, Gregor, 06120 Halle/Saale (DE); BOSSE-DOENECKE, Eva, 06120 Halle/Saale (DE)
(74) Representative: Grünecker Patent- und Rechtsanwälte PartG mbB

(57) **Abstract**

The present invention relates to new hyaluronic acid binding proteins. The invention refers to new engineered fusion proteins for use in treating disorders of the eye or the joints. The fusion proteins comprise a subunit that specifically binds to hyaluronic acid. In addition to the hyaluronic acid binding protein, the new fusion proteins comprise a protein therapeutically effective in diseases of the eye or of joints. The fusion proteins are particularly well-suited for medical applications that require extended half-life of eye specific therapeutic proteins.

## Description

### FIELD OF THE INVENTION

The present invention relates to new hyaluronic acid binding proteins. The invention refers to new engineered fusion proteins for use in treating disorders of the eye or the joints. The fusion proteins comprise a subunit that specifically binds to hyaluronic acid. In addition to the hyaluronic acid binding protein, the new fusion proteins comprise a protein therapeutically effective in diseases of the eye or of joints. The fusion proteins are particularly well-suited for medical applications that require extended half-life of eye specific therapeutic proteins.

### BACKGROUND OF THE INVENTION

Disorders of the eye are common diseases affecting many individuals.

Neovascular eye diseases such as for example neovascular (wet) age-related macular degeneration (AMD) are the leading cause of irreversible vision loss among the aging population and affect more than 4 million individuals in the developed countries. AMD causes damage to the macula which is a spot centrally located on the retina of the eye and results in loss of sharp, central vision. Other retinal neovascular diseases include diabetic macular edema (DME), diabetic retinopathy (DR), and macular edema following retinal vein occlusion (RVO). In diabetic retinopathy (DR), a common complication in diabetes, damaged blood vessels of the light-sensitive tissue at the back of usually both eyes are the reason for impaired vision or even vision loss. After the development of diabetic retinopathy patients may be affected by diabetic macular edema where fluids accumulate in the macula of the retina due to leaking blood vessels. Retinal vein occlusion is an eye disease where veins in the retina are blocked resulting in blurry vision or sudden blindness.

In addition to neovascular diseases, there are other significant ocular diseases with unmet therapeutic needs. Geographic atrophy (GA), an advanced form of dry AMD, leads to the progressive degeneration of the retinal pigment epithelium (RPE) and the underlying photoreceptors, resulting in a gradual loss of vision. While there are now approved treatments like complement inhibitors Avacincaptad pegol and Pegcetacoplan, which can slow the progression of GA, the need for more effective and long-lasting treatments remains.

Uveitis, an inflammatory disease affecting the uvea and other parts of the eye, presents another challenge. It can lead to severe complications such as cataracts, glaucoma, and macular edema, which can cause permanent vision loss. The current treatment strategies often involve corticosteroids and immunosuppressive agents, which have systemic side effects and can lead to complications such as increased intraocular pressure and cataract formation. Therefore, there is a substantial need for safer, more effective localized therapies that can provide sustained control of inflammation with minimal side effects.

In addition to retinal diseases, there are other ocular conditions that present significant therapeutic challenges. Neurotrophic keratitis (NK) is a degenerative corneal disease characterized by impaired corneal sensitivity and poor healing, leading to persistent epithelial defects, corneal ulcers, and even perforation. This condition often results from damage to the trigeminal nerve due to herpes simplex or herpes zoster infections, diabetes, or surgical procedures. The treatment landscape for NK includes the use of recombinant human nerve growth factor (Cenegermin) and other protein and peptide biologics currently in development. Cenegermin promotes corneal healing by stimulating the growth and survival of corneal epithelial cells and nerve fibers. However, it must be administered topically as eye drops up to six times a day for an extended period, which can be cumbersome and challenging for patients to adhere to. Other biologics in development also face similar dosing challenges, necessitating frequent administration to maintain therapeutic efficacy.

Thyroid eye disease (TED), also known as Graves' orbitopathy, is an autoimmune inflammatory disorder that affects the orbit around the eye, leading to symptoms such as proptosis (eye bulging), diplopia (double vision), and discomfort. The disease can significantly impair the quality of life and visual function of affected individuals. Teprotumumab, a monoclonal antibody targeting the insulin-like growth factor-1 receptor (IGF-1R), has shown substantial efficacy in reducing proptosis and improving other signs and symptoms of TED. However, Teprotumumab is administered systemically through intravenous infusions every three weeks over a six-month period. While this treatment provides meaningful improvements, the systemic administration is inconvenient and exposes the entire body to the drug, potentially increasing the risk of systemic side effects. This mode of delivery is less than ideal for a condition localized to the periocular region, underscoring the need for more targeted and patient-friendly therapeutic approaches. Ocular surface squamous neoplasia (OSSN) is a malignant condition affecting the conjunctiva and cornea. Interferon alpha-2a (IFNα-2a) is used as a topical treatment to manage OSSN. This cytokine has antiviral, antiproliferative, and immunomodulatory effects, making it effective against neoplastic cells. However, treatment requires frequent application, often multiple times per day, which can be burdensome for patients and poses challenges for adherence.

Dry eye disease (DED) is a multifactorial disease of the ocular surface characterized by loss of homeostasis of the tear film, leading to discomfort, visual disturbance, and potential damage to the ocular surface. Cyclosporine A and Lifitegrast are immunomodulatory agents used to reduce inflammation associated with DED. Both medications are administered as eye drops, often requiring twice-daily dosing. While effective, the need for regular application can be inconvenient for patients, highlighting the need for treatments with prolonged effects or reduced dosing frequency.

Proliferative vitreoretinopathy (PVR) is a complication of retinal detachment surgery characterized by the growth and contraction of membranes within the eye, which can lead to retinal re-detachment. Recombinant platelet-derived growth factor-BB (PDGF-BB) has shown promise in targeting the cellular mechanisms underlying PVR. However, the therapeutic potential of PDGF-BB is limited by the need for repeated intravitreal injections, which are invasive and pose risks of complications.

Retinoblastoma is a rare but life-threatening childhood eye cancer. Melphalan, an alkylating agent, is used in intra-arterial chemotherapy to treat retinoblastoma by directly delivering high concentrations of the drug to the tumor site. Despite its effectiveness, the invasive nature of intra-arterial administration and the potential for systemic toxicity remain significant challenges, underscoring the need for less invasive and more targeted therapeutic strategies.

Atopic keratoconjunctivitis (AKC) is a chronic inflammatory disease affecting the cornea and conjunctiva, often associated with severe itching, redness, and tearing. Tacrolimus, an immunosuppressive agent, is used topically to manage AKC. While effective, it requires regular application, and the potential for local irritation or systemic absorption presents additional challenges for long-term management.

Symptomatic vitreomacular adhesion (VMA) can lead to visual distortion and loss of vision. Ocriplasmin, a proteolytic enzyme, is used as an intravitreal injection to induce pharmacologic vitreolysis, releasing the adhesion and improving visual outcomes. However, the need for intravitreal injection and the potential for side effects such as transient vision loss or retinal tears highlight the need for improved delivery methods and safer alternatives.

The vitreous of the eye contains as major protein components collagen type II and albumin and as major carbohydrate constituent hyaluronic acid. Hyualuronic acid increases with age and is an ideal target for therapeutic proteins for eye diseases.

Current procedures for the treatment of eye diseases such as AMD include proteins like Aflibercept, Ranibizumab, or Bevacizumab. Although a topical application of these drugs is possible, a disadvantage of the topical application is a very fast wash out from the eye and accordingly, a very low fraction of drug uptake. An alternative to topical applications to the eye are injections; however, the treatment of eye diseases requires frequent intraocular injections of drugs as often as every 1-2 months, into one or both eyes, often for life. This sums up to 6-12 injections per year. Pharmaceutical interventions through injections into the vitreous are inconvenient for patients since they are risky and painful for the patients.

Thus, needless to say that existing therapies for eye diseases have significant disadvantages for patients. They are burdensome for patients in terms of pain, cost, time, and risk. There is a strong need to relief patients from the imperative frequent and burdening treatments of eye diseases. Due to significant limitations of current therapies for eye diseases, there was a need to provide novel proteins for the treatment of ocular diseases with improved properties, in particular in view of prolonged availability in the eye to reduce the painful frequent injection. Accordingly, there is a need in this field to obtain novel proteins suitable for more effective approaches for the therapy of eye diseases.

One objective of the present invention is the provision of molecules for anchoring a therapeutic protein effective for eye diseases near the diseased ocular tissue to prolong the beneficial impact of the therapeutic protein for eye diseases.

The present invention provides artificial fusion proteins for a therapeutic protein effective for eye diseases and hyaluronic acid binding proteins that are particularly well-suited for the treatment of eye diseases but overcome the disadvantages of current approaches. Hyaluronic acid is a high-molecular-mass polysaccharide found in the extracellular matrix, especially of soft connective tissues.

The above-described objectives and advantages are achieved by the subject-matters of the enclosed claims. The present invention meets the needs presented above by providing examples for fusion proteins. Preferred embodiments of the invention are included in the claims as well as in the following description, examples and figures. The above overview does not necessarily describe all problems solved by the present invention.

### SUMMARY OF THE INVENTION

The present disclosure provides the following items 1 to 15, without being specifically limited thereto:
[1] A hyaluronic acid binding protein comprising an amino acid sequence having at least 80 % or at least 85 % sequence identity to any one of SEQ ID NO: 1, SEQ ID NO: 2, or SEQ ID NO: 3.
[2] A hyaluronic acid binding protein comprising an amino acid sequence with at least 80 % or at least 85 % sequence identity to SEQ ID NO: 3 or SEQ ID NO: 4 wherein the protein has (i) a proline (P) at the position corresponding to position 8 in SEQ ID NO: 3 or SEQ ID NO: 4, (ii) a glycine (G) at the position corresponding to position 13 in SEQ ID NO: 3 or SEQ ID NO: 4, and (iii) a glutamine (Q) at the position corresponding to position 40 in SEQ ID NO: 3 or SEQ ID NO: 4. As described elsewhere herein, the hyaluronic acid binding protein exhibits advantageous properties against proteolytic degradation. In particular, hyaluronic acid binding proteins of the present invention show improved stability against proteolytic degradation as compared to the same proteins not comprising (i) a proline (P) at the position corresponding to position 8 in SEQ ID NO: 3 or SEQ ID NO: 4, (ii) a glycine (G) at the position corresponding to position 13 in SEQ ID NO: 3 or SEQ ID NO: 4, and (iii) a glutamine (Q) at the position corresponding to position 40 in SEQ ID NO: 3 or SEQ ID NO: 4.
[3] The hyaluronic acid binding protein of item [1] or [2], wherein the protein has a specific binding affinity for hyaluronic acid of at least 100 nM. As described elsewhere herein, this is considered to be equivalent to the hyaluronic acid binding protein having a specific binding affinity for hyaluronic acid of 100 nM or less. In various embodiments, the specific binding affinity is less than100 nM.
[4] A fusion protein comprising
   a) a hyaluronic acid binding protein according to item [1] or item [2], and
   b) a therapeutic protein, preferably for the treatment of eye diseases or for the treatment of diseases of the joints.
[5] The fusion protein of item [4], wherein the therapeutic protein is a monoclonal antibody, fusion protein, recombinant protein, peptide drug, growth factor, immunosuppressant, enzyme, or protein inhibitor. As described elsewhere herein, in various embodiments of the present invention, the fusion protein comprises an amino acid sequence with at least 80 % sequence identity to SEQ ID NO: 7 or SEQ ID NO: 8. In various embodiments, the fusion protein comprising an amino acid sequence with at least 80 % sequence identity to SEQ ID NO: 7 or SEQ ID NO: 8 comprises a therapeutic protein comprising an amino acid sequence with at least 80 %, preferably at least 85 %, more preferably at least 90 % or even 95 %, sequence identity to SEQ ID NO: 9. Preferably, the therapeutic protein comprising an amino acid sequence with at least 80 % sequence identity to SEQ ID NO: 9 as described above exhibits any of the functional properties of Aflibercept as described elsewhere herein. Accordingly, the present invention encompasses embodiments directed to fusion proteins comprising an amino acid sequence with at least 80 % sequence identity to SEQ ID NO: 7 or SEQ ID NO: 8, wherein the fusion protein comprises a therapeutic protein, which comprises an amino acid sequence with at least 80 % sequence identity to SEQ ID NO: 9, and which exhibits any, in particular one or more, of the functional properties of Aflibercept as described elsewhere herein. Preferably, the therapeutic protein comprising an amino acid sequence with at least 80 % sequence identity to SEQ ID NO: 9 exhibits all of the functional properties of Aflibercept as described elsewhere herein.
[6] The fusion protein of item [4], wherein the therapeutic protein for the treatment of eye diseases is selected from Aflibercept, Ranibizumab, Bevacizumab, Brolucizumab, Farcimab, Adalimumab, Infliximab, Teprotumumab, Cenegermin, Interferon alpha-2a, Cyclosporine A, Lifitegrast, recombinant human platelet-derived growth factor (PDGF-BB), Melphalan, Tacrolimus, Mycophenolate Mefetil, Ocriplasmin, Anakinra, Toclizumab, Pegcetacoplan, Avacincaptad peptol, or derivatives of any of those, or biosimilars.
[7] The fusion protein of item [4], wherein the fusion protein comprises two subunits that bind to hyaluronic acid and to a disease target, respectively, wherein the first subunit comprises a hyaluronic acid binding protein according to item [1] or [2], and wherein the second subunit is a therapeutic protein.
[8] The fusion protein of item [4] for use in the treatment of eye diseases.
[9] The fusion protein of item [8], wherein the fusion protein has at least 1.5 fold higher concentration in the vitreous body 4 days after application (administration) as compared to the concentration of the therapeutic protein under the same conditions. Preferably, the fusion protein has at least 3-fold higher concentration 14 days after application (administration) as compared to the therapeutic protein. More preferably, the fusion protein has at least 5-fold higher concentration 21 days after application (administration) as compared to the therapeutic protein.
   In various embodiments of the fusion protein for use according to item [8], the half-life of the therapeutic protein is at least 1.5-fold longer than the half-life of the therapeutic protein without the hyaluronic acid binding protein.
[10] The fusion protein of item [8], wherein the fusion protein has at least 30% longer half-life compared to the therapeutic protein, in particular wherein the half-life means vitreal half-life (i.e., half-life in the vitreous body).
[11] A composition comprising the hyaluronic acid binding protein of item [1], or the fusion protein of item [4], for delivery to the eye or the joints.
[12] A composition comprising the fusion protein according of item [4] and a therapeutically acceptable carrier and/or diluent for the treatment of eye diseases or diseases of the joints.
[13] A composition comprising the hyaluronic acid binding protein of item [1] or item [2] and a diagnostically acceptable carrier and/or diluent for the diagnosis of eye diseases or diseases of the joints.
[14] A method for treating an eye disease, the method comprising administering to the eye of a subject in need thereof a therapeutically effective amount of the fusion protein of item [4], or the composition of item [10], in an amount and via a route sufficient to treat the eye disease.
[15] A method for producing a hyaluronic acid binding protein according to item [1], or a fusion protein of item [4], comprising the steps of a) culturing of a host cell comprising a polynucleotide encoding the hyaluronic acid protein according to item [1], or the fusion protein of item [4], under conditions suitable to obtain said protein and b) isolating said protein produced from the host cell or from a medium in which the host cell was cultured.

This summary of the invention does not necessarily describe all features of the present invention. Other embodiments become apparent from a review of the ensuing detailed description.

### BRIEF DESCRIPTION OF THE FIGURES.

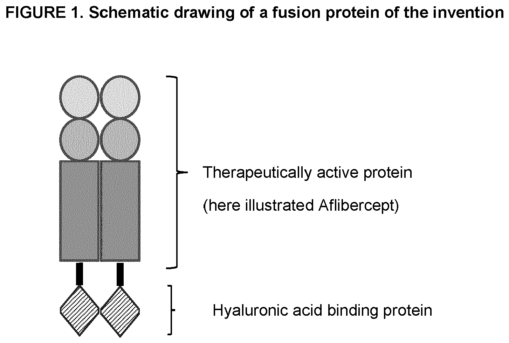

**Figure 1****.** shows a schematic drawing of a fusion protein of the invention. The schematic drawing refers to Aflibercept as therapeutic protein. Circles show domains of the extracellular domains of VEGFR1 and VEGFR2, respectively, rectangle shows the Fc part of a human IgG1, black line refers to a linker, diamonds with lines show the hyaluronic acid binding protein domains.

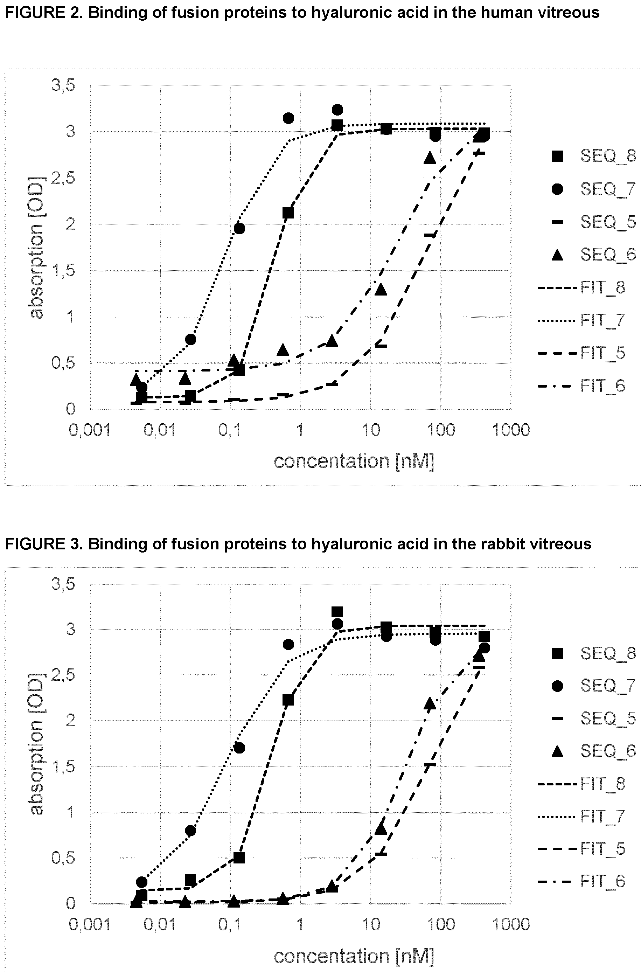

**Figure 2** shows that fusion proteins bind to hyaluronic acid in the human vitreous. Shown is the K_{D}-determination of fusion proteins by ELISA. The black square refers to the fusion protein having the amino acid sequence of SEQ ID NO: 8 (comprising SEQ ID NO: 4), the black circle refers to the fusion protein having the amino acid sequence of SEQ ID NO: 7 (comprising SEQ ID NO: 3), the line) refers to the fusion protein having the amino acid sequence of SEQ ID NO: 5 (comprising SEQ ID NO: 1), and the black flilled rectangle refers to the fusion protein having the amino acid sequence of SEQ ID NO: 6 (comprising SEQ ID NO: 2). Lines represent best fits to a four-parameter logistic binding model.
**Figure 3** shows that fusion proteins bind to hyaluronic acid in the rabbit vitreous. Shown is the KD-determination of fusion proteins by ELISA. Lines represent best fits to a four-parameter logistic binding model.

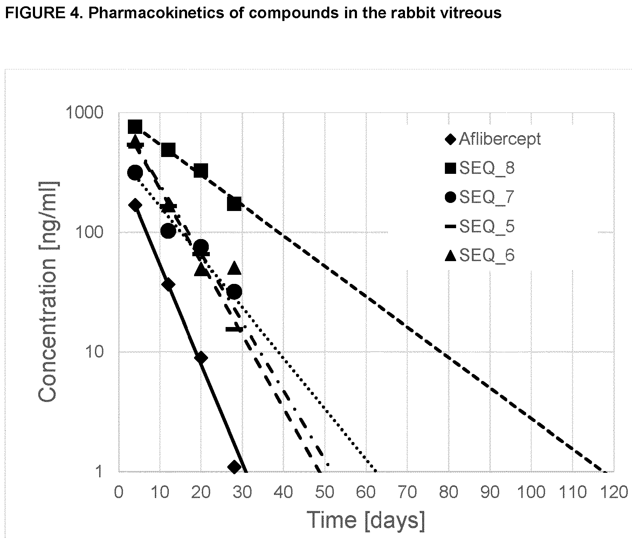

**Figure 4** shows the pharmacokinetic profile of the four fusion proteins binding to hyaluronic acid. Measurements were taken at day 4, 12, 20 and 28. Concentrations in the vitreous were determined by ELISA. Lines represent best fits to a single exponential decay.

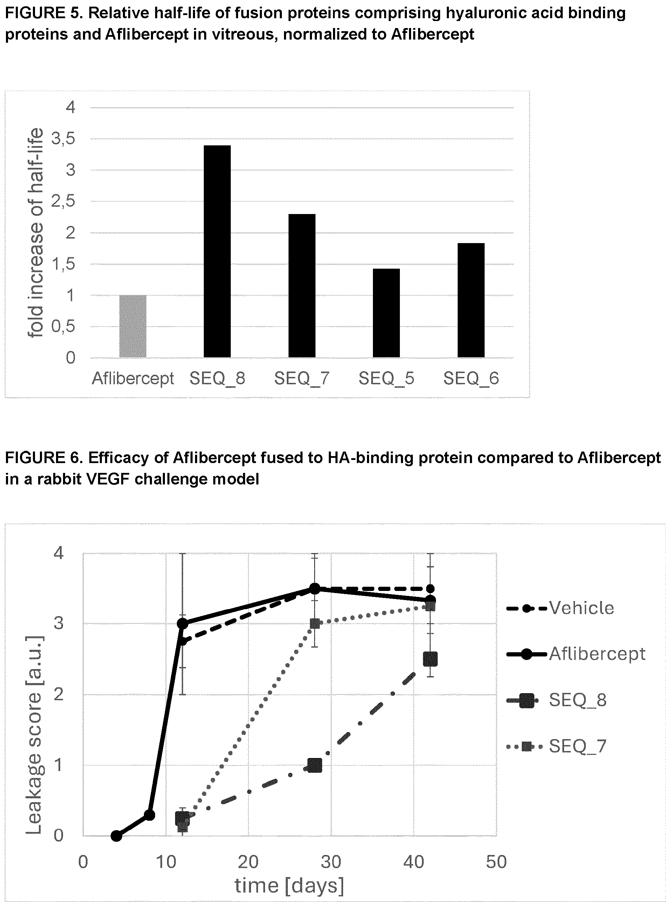

**Figure 5** shows the relative half-lifes of the hyaluronic acid binding proteins obtained by a single-exponential fit to the data in **Figure 4** and normalized to Aflibercept.
**Figure 6** shows the leakage score of the hyaluronic acid binding proteins and Aflibercept. The black square refers to the fusion protein having the amino acid sequence of SEQ ID NO: 8 (comprising SEQ ID NO: 4), the small black squares and dotted line refers to the fusion protein having the amino acid sequence of SEQ ID NO: 7 (comprising SEQ ID NO: 3),

### DETAILED DESCRIPTION OF THE INVENTION

The proteins of the invention bind to hyaluronic acid. The fusion proteins of the invention bind to the major component of the vitreous body of the eye, hyaluronic acid. The other part of the novel fusion protein or fusion polypeptide of the invention is a therapeutic protein effective in the therapy of eye diseases, including treatment and prevention of eye diseases. Due to the anchoring of the fusion protein in the eye by binding to hyaluronic acid, the local residence time of the therapeutic protein is enhanced so that less frequent painful treatments of eye diseases are required. The novel hyaluronic acid binding proteins and fusion proteins of the invention enable fewer medical interventions and safer therapies in eye diseases and improve quality of life for patients.

The present inventors have developed a solution to meet the ongoing need in the art by providing fusion proteins comprising hyaluronic acid specific proteins and a therapeutic protein effective in the treatment of eye diseases or joint diseases. The hyaluronic acid specific proteins are functionally characterized by affinity for hyaluronic acid. The fusion proteins of the invention provide molecular formats with favorable physicochemical properties, in particular, they are stable and may broaden therapeutic options. Therapeutic proteins for eye diseases, if fused to the hyaluronic acid binding protein as disclosed herein, may have a longer duration of action in eye diseases since the clearance of the therapeutic protein from the vitreous body is decreased and thereby the half-life is extended. The fusion proteins provided herein, the compositions and methods allow for retention of therapeutic proteins for eye diseases for a longer period of time.

Further, this enhances patient acceptance and quality of life and is an improvement over current treatment strategies. Therapeutic proteins for joint diseases, if fused to the hyaluronic acid binding protein as disclosed herein, may have a longer duration of action in joint diseases since the clearance of the therapeutic protein is decreased and thereby the half-life is extended.

Before the present invention is described in more detail below, it is to be understood that this invention is not limited to the particular methodology, protocols and reagents described herein as these may vary. It is also to be understood that the terminology used herein is for the purpose of describing particular aspects and embodiments only and is not intended to limit the scope of the present invention, which is reflected by the appended claims. Unless defined otherwise, all technical and scientific terms used herein have the same meaning as commonly understood by one of ordinary skill in the art to which this invention belongs. This includes a skilled person working in the field of protein engineering and purification, but also including a skilled person working in the field of developing new fusion molecules for use in therapy of various eye diseases. Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims, which follow, unless the context requires otherwise, the word "comprise", and variants such as "comprises" and "comprising", was understood to imply the inclusion of a stated integer or step, or group of integers or steps, but not the exclusion of any other integer or step or group of integers or steps. The term "comprise(s)" or "comprising" may encompass a limitation to "consists of" or "consisting of", should such a limitation be necessary for any reason and to any extent.

Several documents (for example: patents, patent applications, scientific publications, manufacturer's specifications, instructions, GenBank Accession Number sequence submissions etc.) may be cited throughout the present specification. Nothing herein is to be construed as an admission that the invention is not entitled to antedate such disclosure by virtue of prior invention. All sequences referred to herein are disclosed in the attached sequence listing that, with its whole content and disclosure, forms part of the disclosure content of the present specification.

### GENERAL DEFINITIONS OF IMPORTANT TERMS USED IN THE APPLICATION

The terms "hyaluronic acid binding protein" or "protein with binding specificity for hyaluronic acid" refers to a protein capable of binding to hyaluronic acid.

The term "fusion protein" relates to a protein comprising at least a first protein joined genetically to at least a second protein. A fusion protein is created through joining of two or more genes that originally coded for separate proteins. Fusion proteins may further comprise additional domains that are not involved in binding of the target, such as but not limited to, for example, multimerization moieties, polypeptide tags, polypeptide linkers, half-life extending moieties.

The terms "protein" and "polypeptide" refer to any chain of two or more amino acids linked by peptide bonds and does not refer to a specific length of the product. Thus, "peptides", "protein", "amino acidchain", or any other term used to refer to a chain of two or more amino acids, are included within the definition of "protein", and the term "protein" may be used instead of, or interchangeably with, any of these terms. The term "protein" is also intended to refer to the products of post-translational modifications of the polypeptide which are well known in the art. The terms "therapeutic protein for eye diseases" or "protein therapeutically effective in eye diseases" may be interchangeably and relate to a protein that is used for therapies of eye diseases. Thus, a therapeutic protein for eye diseases is understood as a protein for use in treating a disorder that affects the eye. Therapeutic proteins for eye diseases might be classified based on pharmacological action, eg. group I: protein therapeutics with enzymatic or regulatory activity (replacement of a protein that is deficient or abnormal; augmentation or inhibition of an existing pathway; provides a novel function or activity); group II: protein therapeutics with special targeting activity (interferes with a molecule or signaling pathway, delivers other compounds or proteins such as radionuclide, cytotoxic drug, or effector protein). Another classification of therapeutic proteins is based on molecular types: antibody based binders, non-immunoglobulin scaffold based binders, enzymes, growth factors, hormones, interferons, and interleukins as well as fusions of these such as Fc-fusions or fusions with half-life extending moieties. Yet a further classification is based on molecular mechanism: binding non-covalently to target (antibodies and non-immunoglobulin scaffolds and others), pathway activation or pathway inhibition, or enzymatic or transport or structural.

A therapeutic protein effective in the treatment of eye diseases may be used for the treatment or prevention of neovascular (wet) age-related macular degeneration (AMD), diabetic macular edema (DME), diabetic retinopathy (DR), macular edema following retinal vein occlusion (RVO), non-infectious uveitis, thyroid eye disease, neurotrophic keratitits, ocular surface squamous neoplasia, dry eye disease, proliferative vitreoretinopathy, retinoblastoma, atopic keratoconjunctivitis, vitreomacular adhesion, myopic choroidal neovascularization, and other. The term "VEGF" or "vascular endothelial growth factor" is a human vascular endothelial growth factor. VEGF-A (uniprot Accession Number P15692) exists as a number of different isotypes which are generated both by alternative splicing and proteolysis, for example, VEGF-206, VEGF-189, VEGF-165, and VEGF-121. The isoforms are all biologically active as dimers. VEGF-A herein means any of the natural isoforms or natural variants or induced variants having at least a sequence identity of at least 80 %, 85 %, 90 %, 95 %, 96 % or 97 % or more, or 100 % to a natural isoform or natural variant. In some embodiments, VEGF-A is human VEGF-A.

The terms "protein with binding specificity for VEGF-A" or "VEGF-A binding protein" or "VEGF-A specific binding protein" refer to a protein with high affinity binding to VEGF-A.

The term "modification" or "amino acid modification" refers to a substitution, a deletion, or an insertion of an amino acid at a particular position in a parent polypeptide sequence by another amino acid. Given the known genetic code and recombinant and synthetic DNA techniques the skilled scientist can readily construct DNAs encoding the amino acid variants.

The term "substitution" is understood as exchange of an amino acidby another amino acid. The term "insertion" comprises the addition of amino acids to the original amino acid sequence.

The terms "binding affinity" and "binding activity" may be used herein interchangeably, and they refer to the ability of a polypeptide of the invention to bind to another polysaccharide, protein, peptide, or fragment or domain thereof. Binding affinity is typically measured and reported by the equilibrium dissociation constant (K_{D}), which is used to evaluate and rank order strengths of bimolecular interactions.

The term "amino acid sequence identity" refers to a quantitative comparison of the identity (or differences) of the amino acid sequences of two or more proteins. "Percent (%) amino acid sequence identity" with respect to a reference polypeptide sequence is defined as the percentage of amino acid residues in a sequence that are identical with the amino acid residues in the reference polypeptide sequence, after aligning the sequences and introducing gaps, if necessary, to achieve the maximum percent sequence identity. To determine the sequence identity, the sequence of a query protein is aligned to the sequence of a reference protein or polypeptide, for example, to the amino acid sequence of the polypeptide of SEQ ID NO: 1. Methods for sequence alignment are well known in the art. For example, the NCBI BLAST similarity program is preferably employed (Camacho et al. 2009, BMC Bioinformatics 10: 421). For multiple alignment analysis, ClustalL is preferably used (Sievers & Higgins, 2021, Methods Mol Biol. 2231: 3-16). Thus, the percentage of an amino acid sequence identity may be calculated using a published or commercially available software with an algorithm which conducts comparison using a base sequence (e.g., SEQ ID NO: 1 in the present invention) as a reference sequence. For example, BLAST, FASTA, or GENETYX (manufactured by Software Development Co., Ltd.) may be used, and these may be run with default parameters. Each amino acid of the query sequence that differs from the aligned reference amino acid sequence at a given position is counted as one difference. An insertion or deletion in the query sequence is also counted as one difference. The sum of differences is then related to the length of the aligned reference sequence to yield a percentage of non-identity or identity, respectively.

The term "half-life" refers to the time that is needed for the concentration of a therapeutic protein for eye diseases to be reduced by one-half.

### DETAILED DESCRIPTION OF THE EMBODIMENTS OF THIS INVENTION

The invention relates to a hyaluronic acid binding protein comprising an amino acid sequence with at least 80 % or at least 85 %, preferably at least 90 % sequence identity to SEQ ID NO: 1, SEQ ID NO: 2, or SEQ ID NO: 3.

The invention relates to hyaluronic acid binding protein comprising an amino acid sequence with at least 80 % or at least 85 %, preferably at least 90 % sequence identity to SEQ ID NO: 3 wherein the protein has (i) a proline (P) at the position corresponding to position 8 in SEQ ID NO: 3, (ii) a glycine (G) at the position corresponding to position 13 in SEQ ID NO: 3, and (iii) a glutamine (Q) at the position corresponding to position 40 in SEQ ID NO: 3.

The invention relates to hyaluronic acid binding protein comprising an amino acid sequence with at least 80 % or at least 85 %, preferably at least 90 % sequence identity to SEQ ID NO: 4 wherein the protein has (i) a proline (P) at the position corresponding to position 8 in SEQ ID NO: 4, (ii) a glycine (G) at the position corresponding to position 13 in SEQ ID NO: 4, and (iii) a glutamine (Q) at the position corresponding to position 40 in SEQ ID NO: 4.

Amino acids in position 8, 13, and 40 of SEQ ID NO: 3 or SEQ ID NO: 4 are important for protease stability of the hyaluronic acid binding protein of SEQ ID NO: 3 or SEQ ID NO: 4 or variants thereof.

The term "protease stable" refers herein to the hyaluronic acid binding protein with improved stability against proteolytic degradation. A protease stable hyaluronic acid binding protein is less prone to proteolysis. Protease stability thus refers to the amount of the uncleaved hyaluronic acid binding protein remaining after exposure to protease. The concentration of a protease stable hyaluronic acid binding protein after exposure to protease is at least about 2fold, 3fold, 4fold higher than for a protease unstable hyaluronic acid binding protein. Protease stability can be determined by measuring the concentration of the uncleaved hyaluronic acid binding protein after exposure to a protease for a period of time sufficient to provide cleavage of the protein into smaller peptide fragments.

The invention relates to hyaluronic acid binding protein comprising an amino acid sequence with at least 80 % or at least 85 %, preferably at least 90 % sequence identity to SEQ ID NO: 3, wherein the protein has (i) a proline (P) at the position corresponding to position 8 in SEQ ID NO: 3, (ii) a glycine (G) at the position corresponding to position 13 in SEQ ID NO: 3, and (iii) a glutamine (Q) at the position corresponding to position 40 in SEQ ID NO: 3, provided that the hyaluronic acid binding protein is protease stable. As described (elsewhere) herein, a hyaluronic acid binding protein of the present invention is protease stable as it is less prone to proteolysis. In particular, a hyaluronic acid binding protein of the present invention is protease stable to the effect that it shows improved stability against proteolytic degradation as compared to the same protein not comprising (i) a proline (P) at the position corresponding to position 8 in SEQ ID NO: 3, (ii) a glycine (G) at the position corresponding to position 13 in SEQ ID NO: 3, and (iii) a glutamine (Q) at the position corresponding to position 40 in SEQ ID NO: 3. As will be appreciated by a person of ordinary skill in the art, (improved) stability against proteolysis or stability against proteolytic degradation, means proteolysis or proteolytic degradation by a proteolytic enzyme, a protease. In various embodiments, (improved) stability against proteolysis or stability against proteolytic degradation in the context of the present invention means proteolysis or proteolytic degradation by a protease such as trypsin, preferably the protease is trypsin. In various embodiments of the present invention, the said improved (increased) stability against degradation by a protease such as trypsin means an improved (increased) stability against degradation by a protease such as trypsin as determined in PBS buffer (preferably determined at 37°C).

In some embodiments, the hyaluronic acid binding protein comprises an amino acid sequence with at least 80 % or at least 85 %, preferably at least 90 %, sequence identity to SEQ ID NO: 3, wherein the protein has (i) a proline (P) at the position corresponding to position 8 in SEQ ID NO: 3, (ii) a glycine (G) at the position corresponding to position 13 in SEQ ID NO: 3, and (iii) a glutamine (Q) at the position corresponding to position 40 in SEQ ID NO: 3, and is at least about 2fold, such as 1.7fold or 1.8fold or 1.9fold, more stable against degradation by a protease such as trypsin than the same protein not having (i) a proline (P) at the position corresponding to position 8 in SEQ ID NO: 3, (ii) a glycine (G) at the position corresponding to position 13 in SEQ ID NO: 3, and (iii) a glutamine (Q) at the position corresponding to position 40 in SEQ ID NO: 3. In some embodiments, the hyaluronic acid binding protein comprises an amino acid sequence with at least 80 % or at least 85 %, preferably at least 90 %, sequence identity to SEQ ID NO: 3, wherein the protein has (i) a proline (P) at the position corresponding to position 8 in SEQ ID NO: 3, (ii) a glycine (G) at the position corresponding to position 13 in SEQ ID NO: 3, and (iii) a glutamine (Q) at the position corresponding to position 40 in SEQ ID NO: 3, and is at least about 2fold, such as (at least) 1.7fold or (at least) 1.8fold or (at least) 1.9fold, more stable against degradation by a protease such as trypsin as determined 10 min after addition of the protease such as trypsin than the same protein not having (i) a proline (P) at the position corresponding to position 8 in SEQ ID NO: 3, (ii) a glycine (G) at the position corresponding to position 13 in SEQ ID NO: 3, and (iii) a glutamine (Q) at the position corresponding to position 40 in SEQ ID NO: 3. In various embodiments of the present invention, the said increased stability against degradation by a protease such as trypsin means an increased stability against degradation by a protease such as trypsin as determined in PBS buffer (preferably at 37°C). Accordingly, a hyaluronic acid binding protein of the present invention comprising an amino acid sequence with at least 80 % sequence identity to SEQ ID NO: 3 is at least about 2fold, such as (at least) 1.7fold or (at least) 1.8fold or (at least) 1.9fold, more stable against degradation by a protease such as trypsin as determined in PBS buffer 10 min after addition of the protease to the PBS buffer (preferably determined at 37°C).

The invention relates to hyaluronic acid binding protein comprising an amino acid sequence with at least 80 % or at least 85 %, preferably at least 90 % sequence identity to SEQ ID NO: 4, wherein the protein has (i) a proline (P) at the position corresponding to position 8 in SEQ ID NO: 4, (ii) a glycine (G) at the position corresponding to position 13 in SEQ ID NO: 4, and (iii) a glutamine (Q) at the position corresponding to position 40 in SEQ ID NO: 4, provided that the hyaluronic acid binding protein is protease stable. As described (elsewhere) herein, a hyaluronic acid binding protein of the present invention is protease stable as it is less prone to proteolysis. In particular, a hyaluronic acid binding protein of the present invention is protease stable to the effect that it shows improved stability against proteolytic degradation as compared to the same protein not comprising (i) a proline (P) at the position corresponding to position 8 in SEQ ID NO: 4, (ii) a glycine (G) at the position corresponding to position 13 in SEQ ID NO: 4, and (iii) a glutamine (Q) at the position corresponding to position 40 in SEQ ID NO: 4. As will be appreciated by a person of ordinary skill in the art, (improved) stability against proteolysis or stability against proteolytic degradation, means proteolysis or proteolytic degradation by a proteolytic enzyme, a protease. In various embodiments, (improved) stability against proteolysis or stability against proteolytic degradation in the context iof the present invention means proteolysis or proteolytic degradation by a protease such as trypsin, preferably the protease is trypsin. In various embodiments of the present invention, the said improved (increased) stability against degradation by a protease such as trypsin means an improved (increased) stability against degradation by a protease such as trypsin as determined in PBS buffer (preferably determined at 37°C).

In some embodiments, the hyaluronic acid binding protein comprises an amino acid sequence with at least 80 % or at least 85 %, preferably at least 90 %, sequence identity to SEQ ID NO: 4, wherein the protein has (i) a proline (P) at the position corresponding to position 8 in SEQ ID NO: 4, (ii) a glycine (G) at the position corresponding to position 13 in SEQ ID NO: 4, and (iii) a glutamine (Q) at the position corresponding to position 40 in SEQ ID NO:4, and is at least about 2fold, such as 1.7fold or 1.8fold or 1.9fold, more stable against degradation by a protease such as trypsin than the same protein not having (i) a proline (P) at the position corresponding to position 8 in SEQ ID NO: 3, (ii) a glycine (G) at the position corresponding to position 13 in SEQ ID NO: 4, and (iii) a glutamine (Q) at the position corresponding to position 40 in SEQ ID NO: 4. In some embodiments, the hyaluronic acid binding protein comprises an amino acid sequence with at least 80 % or at least 85 %, preferably at least 90 %, sequence identity to SEQ ID NO: 4, wherein the protein has (i) a proline (P) at the position corresponding to position 8 in SEQ ID NO: 4, (ii) a glycine (G) at the position corresponding to position 13 in SEQ ID NO: 4, and (iii) a glutamine (Q) at the position corresponding to position 40 in SEQ ID NO: 4, and is at least about 2fold, such as 1.7fold or 1.8fold or 1.9fold, more stable against degradation by a protease such as trypsin as determined 10 min after addition of the protease such as trypsin than the same protein not having (i) a proline (P) at the position corresponding to position 8 in SEQ ID NO: 4, (ii) a glycine (G) at the position corresponding to position 13 in SEQ ID NO: 4, and (iii) a glutamine (Q) at the position corresponding to position 40 in SEQ ID NO: 4.As will be appreciated by a person of ordinary skill in the art, measuring (improved) stability against proteolysis or stability against proteolytic degradation as described herein above (in particular in relation to both SEQ ID NO: 3 and SEQ ID NO: 4) means measuring (improved) stability against proteolysis or stability against proteolytic degradation of a hyaluronic acid binding protein or the present invention against a protein under the same conditions, i.e., against the same hyaluronic acid binding protein not comprising (i) a proline (P) at the position corresponding to position 8 in SEQ ID NO: 3 or SEQ ID NO: 4, (ii) a glycine (G) at the position corresponding to position 13 in SEQ ID NO: 3 or SEQ ID NO: 4, and (iii) a glutamine (Q) at the position corresponding to position 40 in SEQ ID NO: 3 or SEQ ID NO: 4.

In various embodiments of the present invention, the said increased stability against degradation by a protease such as trypsin means an increased stability against degradation by a protease such as trypsin as determined in PBS buffer (preferably determined at 37°C). Accordingly, a hyaluronic acid binding protein of the present invention comprising an amino acid sequence with at least 80 % sequence identity to SEQ ID NO: 4 is at least about 2fold, such as (at least) 1.7fold or (at least) 1.8fold or (at least) 1.9fold, more stable against degradation by a protease such as trypsin as determined in PBS buffer 10 min after addition of the protease to the PBS buffer (preferably at 37°C).

As described herein, measuring (improved) stability against proteolysis or stability against proteolytic degradation, can be analysed by SDS-PAGE. The SDS-PAGE analysis may be accompanied or performed by spectroscopy, i.e., by using an imaging system or se-HPLC.

As further described herein, the terms "proteolysis", "proteolytic degradation", and "proteolytic cleavage" may be used interchangeably herein.

The hyaluronic acid binding protein may be part of a fusion protein. The fusion protein is comprising a) a hyaluronic acid binding protein with at least 80 % , 85 %, preferably at least 90 % sequence identity to SEQ ID NO: 1, SEQ ID NO: 2, or SEQ ID NO: 3, or with at least 80 %, 85 %, preferably at least 90 % sequence identity to SEQ ID NO: 4 wherein the protein has (i) a proline (P) at the position corresponding to position 8 in SEQ ID NO: 4, (ii) a glycine (G) at the position corresponding to position 13 in SEQ ID NO: 4, and (iii) a glutamine (Q) at the position corresponding to position 40 in SEQ ID NO: 4, and b) a therapeutic protein for the therapy of diseases, preferably for the treatment or prevention of eye diseases or for the treatment or prevention of joint diseases. As described herein, the term therapy includes both treatment and prevention. In the present invention, a preferred aspect is the treatment of eye diseases or diseases of the joints.

The fusion proteins of the invention comprise, essentially consist of, or consist of, at least two subunits wherein the first subunit is a binding protein for hyaluronic acid with at least 80%, 85 %, preferably at least 90 % sequence identity to SEQ ID NO: 1, SEQ ID NO: 2, or SEQ ID NO: 3, or with at least 80 %, 85 %, preferably at least 90 % sequence identity to SEQ ID NO: 4, wherein the protein has (i) a proline (P) at the position corresponding to position 8 in SEQ ID NO: 4, (ii) a glycine (G) at the position corresponding to position 13 in SEQ ID NO: 4, and (iii) a glutamine (Q) at the position corresponding to position 40 in SEQ ID NO: 4, and the second subunit is specific for a therapeutic protein for the treatment or prevention of diseases, preferably eye diseases or diseases of the joint.

In the fusion polypeptides of the present invention, the hyaluronic acid binding protein may be located at the N-terminus or at the C-terminus of the fusion polypeptide. In the fusion polypeptides of the present invention, the hyaluronic acid binding protein may be located at the C-terminus of the fusion polypeptide.

The fusion protein or fusion polypeptide as disclosed herein comprises a therapeutic protein. Preferably, the fusion protein as disclosed herein comprises a therapeutic protein effective in the therapy of eye diseases. In some embodiments, the therapeutic protein can be a selected from, but not limited to, (i) monoclonal antibodies such as Ranibizumab, Bevacizumab, Brolucizumab, Adalimumab, Infliximab, Farcimab, Teprotumumab, or fragments thereof, (ii) fusion proteins such as Aflibercept, (iii) recombinant proteins, such as Cenegermin, Interferon alpha-2a, (iv) peptide drugs such as Cyclosporine A, Lifitegrast,(v) growth factors such as recombinant human platelet-derived growth factor (PDGF-BB), (vi) antimicrobial peptides such as Melphalan, (vii) immunosuppressants such as Tacrolimus, Mycophenolate Mefetil, (viii) enzymes such as Ocriplasmin, (ix) protein inhibitors such as Anakinra or Toclizumab, (x) complement inhibitors such as Pegcetacoplan and Avacincoptad pegol, (xi) other such as Ranibizumab-nuna or Ranibizumab implant, or derivatives of any of those, or biosimilars.

Accordingly, in various embodiments of the present invention, the therapeutic protein is a protein that inhibits the function of a growth factor or the function of a growth factor receptor. Hence, in various embodiments of the present invention, the therapeutic protein is a growth factor inhibitor, or a growth factor receptor inhibitor. More specifically, in various embodiments of the present invention, the therapeutic protein is an endothelial growth factor inhibitor, or an endothelial growth factor receptor inhibitor. In particularly preferred embodiments, the (vascular endothelial) growth factor or (vascular endothelial) growth factor receptor is a (vascular endothelial) growth factor or (vascular endothelial) growth factor receptor associated with a disease or disorder of the eye, in particular .

In the present invention, the therapeutic protein preferably is a protein that inhibits the function of a (vascular endothelial) growth factor like, e.g., VEGF. Aflibercept is a soluble decoy receptor that binds VEGF-A and VEGF-B, and thus exemplifies a preferred therapeutic protein of the present invention.

As described herein, the function of a growth factor, or the function of a growth factor receptor, may be considered as a function in endothelial cell growth or development, in particular vascular endothelial cell growth or development. Accordingly, a therapeutic protein of the present invention may be considered as a (vascular endothelial) growth factor inhibitor that blocks the growth or proliferation of (vascular) endothelial cells. Preferably, a therapeutic protein of the present invention may be considered as a (vascular endothelial) growth factor inhibitor that blocks the growth or proliferation of (vascular) endothelial cells in the eye, in particular retinal endotehial cells.

In some embodiments, the therapeutic protein effective in the treatment of eye diseases is, for example, a vascular endothelial growth factor-A (VEGF-A) binding protein. Accordingly, as described herein, a fusion polypeptide of the invention may comprise a protein with binding specificity for VEGF-A.

In some embodiments, the therapeutic protein effective in the treatment of eye diseases is, for example, a tumor necrosis factor alpha (TNFalpha) binding protein. Accordingly, as described herein, a fusion polypeptide of the invention may comprise a protein with binding specificity for TNFalpha.

In some embodiments, the therapeutic protein effective in the treatment of eye diseases is, for example, a recombinant nerve growth factor (NGF) binding protein. Accordingly, as described herein, a fusion polypeptide of the invention may comprise a protein with binding specificity for NGF.

In various embodiments, the therapeutic protein effective in the treatment of eye diseases may be selected from a monoclonal antibody, fusion protein, recombinant protein, peptide drug, growth factor, immunosuppressant, enzyme, protein inhibitor, or other having binding affinity for a target such as VEGF-A, TNF-alpha, rhNGF, IGF-1R, integrin, IL-1, IL-6.

In various embodiments, the fusion polypeptide comprises an immunoglobulin type disease target specific therapeutic protein such as a VEGF-A specific therapeutic protein, TNF-alpha specific therapeutic protein, or rhNGF specific therapeutic protein. In various preferred embodiments, the immunoglobulin type therapeutic protein is a monoclonal antibody binding to or with specificity to the target such as VEGF-A, TNF-alpha, rhNGF or other. In various preferred embodiments, the antibody binding to or with specificity to the taraget is a full-length antibody, or a fragment thereof. Such antibody fragments include, but are not limited to, single-chain variable fragments (scFv), single-chain antibodies (scAb), and antigen binding frgments (Fab). The full-length antibody binding to or with specificity to VEGF-A comprises an Fc domain.

In various embodiments, the fusion polypeptide as disclosed herein comprises a non-immunoglobulin type target specific therapeutic protein.

In some embodiments, the therapeutic protein effective in the treatment of eye diseases that is directed to VEGF-A as target can be a selected from, but not limited to, Aflibercept, Ranibizumab, Bevacizumab, Brolucizumab, or Farcimab, or fragments thereof, or biosimilars.

In some embodiments, the therapeutic protein effective in the treatment of eye diseases that is directed to TNF-alpha as target can be a selected from, but not limited to, Adalimumab, Infliximab, or fragments thereof, or biosimilars.

In some embodiments, the therapeutic protein effective in the treatment of eye diseases that is directed to rhNGF as target can be a selected from, but not limited to, Cenegermin or fragments thereof, or biosimilars.

In some embodiments the VEGF-A or TNF-alpha or rhNGF specific protein is a therapeutic protein for the treatment of, for example, neovascular eye disease such as neovascular age-related macular degeneration, diabetic macular edema, diabetic retinopathy, macular edema following retinal vein occlusion. In some embodiments the VEGF-A specific protein is a therapeutic protein is selected from but not limited to the VEGF-A specific recombinant monoclonal antibodies Ranibizumab or Bevacizumab or VEGF-specific single-chain antibody fragment Brolucizumab. In some embodiments, the fusion protein comprises Aflibercept as VEGF-A specific protein. In some embodiments, the fusion protein comprises a biosimilar of Aflibercept, for example M710, Mylan NV, ALT-L9, FYB203, CHS-2020, or other VEGF antagonists. Other antagonists could be used instead.

In some embodiments the fusion protein comprises, in addition to the hyaluronic acid binding protein as described herein, a VEGF-A specific protein that is an extracellular domain of a receptor or fragments thereof. In some embodiments VEGF-A specific protein is the recombinant fusion protein Aflibercept (SEQ ID NO: 9).

In various embodiments, the fusion protein comprises (or is fused to, or linked to) the Fc region (or Fc domain) of an immunoglobulin (Ig) molecule. Preferably, the Fc region (or Fc domain) is of a human immunoglobulin (Ig) molecule, more preferably the Fc region (or Fc domain) is of a human IgG molecule, and even more preferably the Fc region (or Fc domain) is of a human IgG1 molecule. In various embodiments, the therapeutic protein of the fusion protein of the invention comprises an Fc region of an immunoglobulin, as described elsewhere herein. Accordingly, disclosed herein are fusion proteins comprising a therapeutic protein, which comprises an Fc region of an immunoglobulin, wherein the fusion protein is linked to or fused to an Fc region (or Fc domain) of an Ig molecule as described above, wherein the Fc region (or Fc domain) is different from the Fc region comprised by the therapeutic protein.

In IgG, IgA and IgD antibody isotypes, the Fc region is composed of two identical protein fragments, derived from the second and third constant domains of the antibody's two heavy chains. Accordingly, the Fc region of an IgG consists of two (paired) CH3 domains and two (separated) CH2 domains.

In various embodiments of the fusion protein of the present invention, the hyaluronic acid binding protein is fused to a therapeutic protein comprising an Fc region as described above. The Fc region typically is an Fc region of a human IgG molecule, preferably of a human IgG1 molecule.

In preferred embodiments of the present invention, the hyaluronic acid binding protein is fused to such therapeutic proteins via the Fc region of the therapeutic protein. More specifically, the hyaluronic acid binding protein is fused to the CH3 domain of the Fc region. Such preferred ambodiments of the present invention are exemplified by SEQ ID NOs: 5-8.

Various preferred embodiments relate to a fusion protein comprising an amino acid sequence of SEQ ID NO: 9, and wherein the fusion protein exhibits specific binding affinity for VEGF-A, in particular for human VEGF-A, in addition to specific binding affinity for hyaluronic acid comprising an amino acid sequence with at least 80 % or 85% sequence identity to SEQ ID NO: 1, SEQ ID NO: 2, or SEQ ID NO: 3 or at least 80 % or 85% sequence identity to SEQ ID NO: **4,** wherein the protein has (i) a proline (P) at the position corresponding to position 8 in SEQ ID NO: 3 or SEQ ID NO: 4, (ii) a glycine (G) at the position corresponding to position 13 in SEQ ID NO: 3 or SEQ ID NO: 4, and (iii) a glutamine (Q) at the position corresponding to position 40 in SEQ ID NO: 3 or SEQ ID NO: 4. Biosimilars of Aflibercept can be used accordingly.

Various embodiments include a therapeutic protein effective in the treatment of eye diseases such as a non-immunoglobulin therapeutic protein. Thus, in some embodiments the fusion protein comprises a) at least 80 %, at least 85 %, preferably at least 90 % identical to any of SEQ ID NOs: 1-4 that bind to hyaluronic acid and b) a therapeutic protein effective in the treatment of eye diseases derived from a non-immunoglobulin scaffold. In some embodiments the fusion protein comprises a therapeutic protein for eye diseases that is an antagonist, for example, based on a non-immunoglobulin effector moiety. In some embodiments a therapeutic protein effective in the treatment of eye diseases may be a VEGF-A specific protein for the treatment of, for example, neovascular eye diseases, for example non-immunoglobulin binding protein Abicipar (Abicipar pegol). Thus, in some embodiments the fusion protein comprises a) any one of SEQ ID NOs: 1-4 that bind to hyaluronic acid of the eye and b) Abicipar.

In some embodiments the fusion protein comprises a) a hyaluronic acid binding protein at least 80 % or at least 85 % identical to SEQ ID NO: 1; and b) a VEGF-antagonist, such as Aflibercept or a biosimilar thereof. A non-limiting example is provided in the amino acid sequence of SEQ ID NO: 5.

In some embodiments the fusion protein comprises a) a hyaluronic acid binding protein at least 80 % or at least 85 % identical to SEQ ID NO: 2; and b) a VEGF-antagonist, such as Aflibercept or a biosimilar thereof. A non-limiting example is provided in the amino acid sequence of SEQ ID NO: 6.

In some embodiments the fusion protein comprises a) a hyaluronic acid binding protein at least 80 % or at least 85 % identical to SEQ ID NO: 3; and b) a VEGF-antagonist, such as Aflibercept or a biosimilar thereof. A non-limiting example is provided in the amino acid sequence of SEQ ID NO: 7.

In some embodiments the fusion protein comprises a) a hyaluronic acid binding protein at least 80 % or at least 85 % identical to SEQ ID NO: 4 wherein the protein has (i) a proline (P) at the position corresponding to position 8 in SEQ ID NO: 4, (ii) a glycine (G) at the position corresponding to position 13 in SEQ ID NO: 4, and (iii) a glutamine (Q) at the position corresponding to position 40 in SEQ ID NO: 4; and b) a VEGF-antagonist, such as Aflibercept or a biosimilar thereof. A non-limiting example is provided in the amino acid sequence of SEQ ID NO: 8.

Some embodiments relate to a fusion protein that comprises an amino acid sequence of of any one of SEQ ID NO: 5, SEQ ID NO: 6, SEQ ID NO: 7, or SEQ ID NO: 8, or an amino acid sequence with at least 80 %, at least 85 %, at least 90 %, or at least 95 % amino acid sequence identity to any one of SEQ ID NOs: SEQ ID NO: 5, SEQ ID NO: 6, SEQ ID NO: 7, or SEQ ID NO: 8.

In some embodiments the fusion protein comprises a) at least 80 %, 85 %, or 90 % identical to any one of SEQ ID NOs: 1-4 that bind to hyaluronic acid of the eye and b) Ranibizumab.

In some embodiments the fusion protein comprises a) at least 80 %, 85 %, or 90 % identical to any one of SEQ ID NOs: 1-4 that bind to hyaluronic acid of the eye and b) Bevacizumab.

In some embodiments the fusion protein comprises a) at least 80 %, 85 %, or 90 % identical to any one of SEQ ID NOs: 1-4 that bind to hyaluronic acid of the eye and b) Brolucizumab.

In some embodiments the fusion protein comprises a) at least 80 %, 85 %, or 90 % identical to any one of SEQ ID NOs: 1-4 that bind to hyaluronic acid of the eye and b) Adalimumab.

In some embodiments the fusion protein comprises a) at least 80 %, 85 %, or 90 % identical to any one of SEQ ID NOs: 1-4 that bind to hyaluronic acid of the eye and b) Infliximab.

In some embodiments the fusion protein comprises a) at least 80 %, 85 %, or 90 % identical to any one of SEQ ID NOs: 1-4 that bind to hyaluronic acid of the eye and b) Faricimab.

In some embodiments the fusion protein comprises a) at least 80 %, 85 %, or 90 % identical to any one of SEQ ID NOs: 1-4 that bind to hyaluronic acid and b) Teprotumumab.

In some embodiments the fusion protein comprises a) at least 80 %, 85 %, or 90 % identical to one of SEQ ID NOs: 1-4 that bind to hyaluronic acid of the eye and b) Cenegermin.

In some embodiments the fusion protein comprises a) at least 80 %, 85 %, or 90 % identical to one of SEQ ID NOs: 1-4 that bind to hyaluronic acid of the eye and b) Interferon alpha-2a.

In some embodiments the fusion protein comprises a) at least 80 %, 85 %, or 90 % identical to any one of SEQ ID NOs: 1-4 that bind to hyaluronic acid of the eye and b) cyclosporine A.

In some embodiments the fusion protein comprises a) at least 80 %, 85 %, or 90 % identical to any one of SEQ ID NOs: 1-4 that bind to hyaluronic acid of the eye and b) Lifitegrast.

In some embodiments the fusion protein comprises a) at least 80 %, 85 %, or 90 % identical to any one of SEQ ID NOs: 1-4 that bind to hyaluronic acid of the eye and b) PDGF-BB.

In some embodiments the fusion protein comprises a) at least 80 %, 85 %, or 90 % identical to any one of SEQ ID NOs: 1-4 that bind to hyaluronic acid of the eye and b) Mephalan.

In some embodiments the fusion protein comprises a) at least 80 %, 85 %, or 90 % identical to any one of SEQ ID NOs: 1-4 that bind to hyaluronic acid of the eye and b) Tacrolinus.

In some embodiments the fusion protein comprises a) at least 80 %, 85 %, or 90 % identical to any one of SEQ ID NOs: 1-4 that bind to hyaluronic acid of the eye and b) Macophenolate Mofetil. In some embodiments the fusion protein comprises a) at least 80 %, 85 %, or 90 % identical to any one of SEQ ID NOs: 1-4 that bind to hyaluronic acid of the eye and b) Ocriplasmin.

In some embodiments the fusion protein comprises a) any one of SEQ ID NOs: 1-4 that bind to hyaluronic acid of the eye and b) Anakinra.

In some embodiments the fusion protein comprises a) at least 80 %, 85 %, or 90 % identical to any one of SEQ ID NOs: 1-4 that bind to hyaluronic acid of the eye and b) Tocilizumab d.

In some embodiments, the fusion polypeptide capable of binding to VEGF-A and hyaluronic acid that comprises at least two subunits, wherein the first subunit is a binding protein specific for VEGF-A is Aflibercept, and the second subunit is a binding protein of at least 80 % or at least 85 %, preferably 90 % identical to any one of SEQ ID NOs: 1-4 specific for hyaluronic acid.

As described herein, it is preferred in the present invention that variants of a hyaluronic acid binding protein that are described herein by percent % identity to any of the amino acid sequences of SEQ ID NOs: 1-4, exhibit the functional properties (in particular binding affinity and prolongation of half-life) of hyaluronic acid binding proteins of the invention as described elsewhere herein. In particular, such (variants of) hyaluronic acid binding proteins that are described herein by percent % identity to any of the amino acid sequences of SEQ ID NOs: 1-4, exhibit the functional properties (in particular with regard to binding affinity and prolongation of half-life as described elsewhere herein) of hyaluronic acid binding proteins as exemplified by fusion proteins of SEQ ID NOs: 5-8, preferably as exemplified by the fusion protein of SEQ ID NO: 8. More preferably, (variants of) hyaluronic acid binding proteins that are described herein by percent % identity to any of the amino acid sequences of SEQ ID NOs: 1-4, exhibit the same functional properties (in particular with regard to binding affinity and prolongation of half-life, as described elsewhere herein) as the hyaluronic acid binding proteins of fusion proteins of SEQ ID NOs: 5-8, preferably of SEQ ID NO: 8.

On other embodiments further components can be included N-terminal and/or C-terminal. Further components may be labels or domains for the purpose of purification or to enhance solubility or for stabilization or for detecting, as known to someone skilled in the art.

Some embodiments of the invention relate to fusion proteins comprising a) a therapeutic protein, preferably for the treatment of eye diseases or for the treatment of diseases of the joints, and b) a hyaluronic acid binding protein, wherein the hyaluronic acid binding protein comprises an amino acid sequence with at least 80 % or at least 85 %, preferably at least 90 % sequence identity to SEQ ID NO: 3 or SEQ ID NO: 4 wherein the hyaluronic acid binding protein has (i) a proline (P) at the position corresponding to position 8 in SEQ ID NO: 3 or SEQ ID NO: 4, (ii) a glycine (G) at the position corresponding to position 13 in SEQ ID NO: 3 or SEQ ID NO: 4, and (iii) a glutamine (Q) at the position corresponding to position 40 in SEQ ID NO: 3 or SEQ ID NO: 4 provided that the hyaluronic acid binding protein is protease stable.

Some embodiments of the invention relate to fusion proteins comprising a) a therapeutic protein, preferably for the treatment of eye diseases or for the treatment of diseases of the joints, and b) a hyaluronic acid binding protein, wherein the hyaluronic acid binding protein comprises an amino acid sequence with at least 80 % or at least 85 %, preferably at least 90 % sequence identity to SEQ ID NO: 3 or SEQ ID NO: 4 wherein the the hyaluronic acid binding protein has (i) a proline (P) at the position corresponding to position 8 in SEQ ID NO: 3 or SEQ ID NO: 4, (ii) a glycine (G) at the position corresponding to position 13 in SEQ ID NO: 3 or SEQ ID NO: 4, and (iii) a glutamine (Q) at the position corresponding to position 40 in SEQ ID NO: 3 or SEQ ID NO:4 and at least about 2fold, such as 1.7fold or 1.8fold or 1.9fold, more stable against degradation by a protease such as trypsin than a protein not having (i) a proline (P) at the position corresponding to position 8 in SEQ ID NO: 3 or SEQ ID NO: 4, (ii) a glycine (G) at the position corresponding to position 13 in SEQ ID NO: 3 or SEQ ID NO: 4, and (iii) a glutamine (Q) at the position corresponding to position 40 in SEQ ID NO: 3 or SEQ ID NO: 4.

Some embodiments of the invention relate to fusion proteins comprising a) a therapeutic protein, preferably for the treatment of eye diseases or for the treatment of diseases of the joints, and b) a hyaluronic acid binding protein, wherein the hyaluronic acid binding protein comprises an amino acid sequence with at least 80 % or at least 85 %, preferably at least 90 % sequence identity to SEQ ID NO: 3 or SEQ ID NO: 4, wherein the protein has (i) a proline (P) at the position corresponding to position 8 in SEQ ID NO: 3 or SEQ ID NO: 4, (ii) a glycine (G) at the position corresponding to position 13 in SEQ ID NO: 3 or SEQ ID NO: 4, and (iii) a glutamine (Q) at the position corresponding to position 40 in SEQ ID NO: 3 or SEQ ID NO: 4, and is at least about 2fold, such as (at least) 1.7fold or (at least) 1.8fold or (at least) 1.9fold, more stable against degradation by a protease such as trypsin as determined 10 min after addition of the protease such as trypsin than a protein not having (i) a proline (P) at the position corresponding to position 8 in SEQ ID NO: 3 or SEQ ID NO: 4, (ii) a glycine (G) at the position corresponding to position 13 in SEQ ID NO: 3 or SEQ ID NO: 4, and (iii) a glutamine (Q) at the position corresponding to position 40 in SEQ ID NO: 3 or SEQ ID NO: 4. In various embodiments of the present invention, the said increased stability against degradation by a protease such as trypsin means an increased stability against degradation by a protease such as trypsin as determined in PBS buffer (preferably determined at 37°C). Accordingly, fusion proteins of the present invention are at least about 2fold, such as (at least) 1.7fold or (at least) 1.8fold or (at least) 1.9fold, more stable against degradation by a protease such as trypsin as determined in PBS buffer 10 min after addition of the protease to the PBS buffer (preferably at 37°C).

Some embodiments relate to the fusion protein that comprises an amino acid sequence of of SEQ ID NOs: 7 or 8, or an amino acid sequence with at least 80 %, at least 85 %, at least 90 %, or at least 95 % amino acid sequence identity to any one of SEQ ID NOs: 7 or 8.

Some embodiments relate to the fusion protein that comprises an amino acid sequence of of SEQ ID NOs: 7 or 8, or an amino acid sequence with at least 80 %, at least 85 %, at least 90 %, or at least 95 % amino acid sequence identity to any one of SEQ ID NOs: 7 or 8, provided that the fusion protein is least about 2fold, such as (at least) 1.7fold or (at least) 1.8fold or (at least) 1.9fold, more stable to degradation by a protease such as trypsin, preferably measured 10 min after the addition of the protease such as trypsin. In various embodiments, the fusion protein is (at least) 1.7 fold more stable to degradation by a protease such as trypsin, preferably measured 10 min after the addition of the protease such as trypsin. In various embodiments of the present invention, the said increased stability against degradation by a protease such as trypsin means an increased stability against degradation by a protease such as trypsin as determined in PBS buffer (preferably determined at 37°C). Accordingly, in various embodiments, the fusion protein is at least about 2fold, such as (at least) 1.7fold or (at least) 1.8fold or (at least) 1.9fold, more stable against degradation by a protease such as trypsin as determined in PBS buffer 10 min after addition of the protease to the PBS buffer (preferably at 37°C).

Linker. In some embodiments the fusion protein or fusion polypetide as defined above or as defined elsewhere herein can include a linker, for example a polypeptide linker, between the hyaluronic acid binding protein and the therapeutic protein.

The length and composition of a linker may vary between at least one and up to about 50 amino acids. More preferably, the peptide linker has a length of between 1 and 30 amino acids; e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acids. It is preferred that the amino acid sequence of the peptide linker is not immunogenic to human beings, stable against proteases and optionally does not form a secondary structure. Suitable amino acids for linkers may be selected but are not limited to from amino acids such as glycine, serine, alanine, or proline. A suitable linker of 15 amino acids (glycine and serine) is shown in SEQ ID NO: 10. The linker of SEQ ID NO: 10 is composed of three (glycine / serine) units. Disclosed herein are suitable linkers that are composed of one or two of these (glycine / serine) units. Further disclosed herein are suitable linkers that are composed of four or more of these (glycine / serine) units.

The proteins bind hyaluronic acid with a measurable binding affinity of less than 100 nM, of less than 50 nM, less than 10 nM, or less than 5 nM. One embodiment refers to a fusion protein or fusion polypeptide of the invention comprising a hyaluronic acid binding protein of the invention with a binding affinity (K_{D}) of less than 100 nM for hyaluronic acid. Further, the fusion proteins bind the target of the therapeutic protein with a measurable binding affinity (K_{D}) of less than 100 nM, of less than 50 nM, less than 10 nM, less than 5 nM, or less than 1 nM. In some embodiments, the fusion protein binds hyaluronic acid with a binding affinity of less than 100 nM and binds the target of the therapeutic protein with binding affinity of less than 50 nM. In other embodiments, the fusion protein binds hyaluronic acid with binding affinity of less than 100 nM and binds the target of the therapeutic protein with binding affinity of less than 10 nM. In some embodiments, the binding affinities (K_{D}) of the fusion protein for hyaluronic acid and for the target of the therapeutic protein are different. The appropriate methods are known to those skilled in the art or described in the literature. The methods for determining the binding affinities are known per se and can be selected for instance from the following methods known in the art: enzyme-linked immunosorbent assay (ELISA), surface plasmon resonance (SPR), kinetic exclusion analysis (KinExA assay), Bio-layer interferometry (BLI), flow cytometry, fluorescence spectroscopy techniques, isothermal titration calorimetry (ITC), analytical ultracentrifugation, radioimmunoassay (RIA or IRMA), and enhanced chemiluminescence (ECL). Some of the methods are described in the Examples below. Typically, the dissociation constant K_{D} (and, hence, the binding affinity) is determined at 20°C, 25°C, or 30°C. The lower the K_{D} value, the greater the binding affinity of the biomolecule for its binding partner. The higher the K_{D} value, the more weakly the binding partners bind to each other.

In various (preferred) embodiments of the present invention, the affinity of the binding protein to hyaluronic acid is a binding affinity as determined via ELISA (Example 3). More preferably, the binding affinity means the binding affinity for hyaluronic acid in human or rabbit virtreous, as determined by ELISA (Example 3). In particularly preferred embodiments, a hyaluronic acid binding protein of the present invention has a binding afficity for hyaluronic acid in the human or rabbit vitreous of even less than 10 nM, as determined by ELISA (SEQ ID NO: 8; Example 3). The fusion protein according to SEQ ID NO: 8 exemplies a particularly preferred embodiments of the present invention. Other preferred embodiments of the present invention are exemplified by of SEQ ID NOs: 5-7.

By specifically binding to hyaluronic acid in the eye, the hyaluronic acid specific binding protein may anchor the therapeutic protein to the fibrillar structure of the vitreous body and thereby ensure an increase of the half-life of the therapeutic protein for eye diseases. In some embodiments the half-life of the therapeutic protein is increased by fusion to a hyaluronic acid binding protein as disclosed herein. Concentration can be measured by methods known to some skilled in the art, and include ELISA, mass spectroscopy, western blot, radio-immunoassay, or fluosecent labeling. Methods for pharmocokinetic analysis and determination of half-life and/or mean residence time are known in the art. **FIGURE 4** measures the concentration of the therapeutic protein or fusion protein comprising the protein of the invention after 4, 12, 20, 28 days. **FIGURE 5** shows the half-life relative to the therapeutic protein. In preferred embodiments, half-life of the therapeutic protein for eye disease is increased at least 3fold 12 days after application, as measured as concentration of the therapeutic protein or fusion protein comprising the hyaluronic acid binding protein of the invention. In further preferred embodiments, the half-life of the therapeutic protein for eye disease is increased at least 5fold 20 days after application, as measured as concentration of the therapeutic protein or fusion protein comprising the protein of the invention. In preferred embodiments, the concentration of the fusion protein comprising the protein of the invention is at least 10 ng/mL 28 days after application to the vitreous humuor. Preferably, the concentration of the therapeutic protein or fusion protein means concentration in the vitreous (vitreous body). More specifically, the concentration of the therapeutic protein or fusion protein means concentration in the vitreous (vitreous body) after intravitreal administration.

Some embodiments relate to the fusion protein as described above for use in the therapy of eye diseases. In the treatment of eye diseases it is of great importance that the half-life of the therapeutic protein is long (e.g. at least 4 days or longer than 4 days). This can be achieved by a use of the fusion protein comprising the therapeutic protein and the hyaluronic acid binding protein of the invention. In some embodiments, the half-life of a fusion protein as described herein longer than 10 days. In other embodiments the half-life of the fusion protein of the invention is between 4-10 days. In other embodiments the half-life of the fusion protein of the invention is between 4-6 days.

In some embodiments, the half-life of a fusion protein as described herein is around 5 days. In some other embodiments, the half-life of a fusion protein as described herein is around 6 days. In some further embodiments, the half-life of a fusion protein as described herein is around 8days. Still further, in some embodiments, the half-life of a fusion protein as described herein is around 11 days.

In some embodiments, the half-life of the fusion protein comprising a therapeutic protein and hyaluronic acid binding protein of the invention is at least about 1.3fold longer compared to the therapeutic protein without hyaluronic acid binding protein (as exemplified by SEQ ID NO: 5). In other embodiments, the half-life of the fusion protein comprising a therapeutic protein and hyaluronic acid binding protein of the invention is at least about 1.7fold longer compared to the therapeutic protein without hyaluronic acid binding protein (as exemplified by SEQ ID NO: 6; Fig. 5). In some embodiments, the half-life of the fusion protein comprising a therapeutic protein and hyaluronic acid binding protein of the invention is at least 2.2fold longer compared to the therapeutic protein without hyaluronic acid binding protein (as exemplified by SEQ ID NO: 7; Fig. 5). In some embodiments, the half-life of the fusion protein comprising a therapeutic protein and hyaluronic acid binding protein of the invention is at least 3.4fold longer compared to the therapeutic protein without hyaluronic acid binding protein (as exemplified by SEQ ID NO: 8; Fig. 5).

In various embodiments, the fusion polypeptide of the invention comprises a target, e.g. VEGF-A, specific binding protein and a hyaluronic acid specific binding protein, wherein the a target, e.g. VEGF-A specific binding protein exhibits any of the functional properties described elsewhere herein for a target, e.g. VEGF-A specific proteins, and wherein the hyaluronic acid specific binding protein exhibits any of the functional properties described elsewhere herein for hyaluronic acid binding proteins. Accordingly, in various embodiments, any of the functional characteristics or properties described herein in relation to a target, e.g. VEGF-A specific binding protein may be combined with any of the functional characteristics or properties described herein in relation to a hyaluronic acid specific binding protein.

In some embodiments, the hyaluronic acid binding protein is a multimer comprising of a plurality of the hyaluronic acid binding protein as defined herein. A multimer may comprise two, three, four, or more hyaluronic acid binding proteins. In one embodiment, the hyaluronic acid binding protein comprises 2, 3, 4, or more hyaluronic acid binding proteins linked to each other, i.e. the hyaluronic acid-binding protein can be a dimer, trimer, or tetramer, etc. In some embodiments, the multimer is a dimer of the hyaluronic acid binding protein as defined above. In some embodiments, the multimeric hyaluronic acid binding protein may comprise at least two modules of proteins 90 % identical to any one of SEQ ID NOs: 1-4. For example, a dimeric hyaluronic acid binding protein may comprise two monomers of SEQ ID NO: 1 or SEQ ID NO: 2 or SEQ ID NO: 3 or SEQ ID NO: 4 linked to each other in head-to-tail orientation.

Multimers of the binding protein are generated artificially, generally by recombinant DNA technology well-known to a skilled person.

**Use in medicine.** Various embodiments relate to the fusion protein as defined above comprising a hyaluronic acid binding protein and a VEGF-A specific protein for use in the treatment or prevention of neovascular eye diseases. Neovascular eye diseases may be selected from the group of but not limited to neovascular (wet) age-related macular degeneration (AMD), diabetic macular edema (DME), diabetic retinopathy (DR), and macular edema following retinal vein occlusion (RVO). Examples for therapeutic proteins for use in the treatment of neovascular eye diseases are Aflibercept, Ranibizumab, Bevacizumab, Faricimab, Brolucizumab, Ranibizumab-nuna, and Ranibizumab Implant.

Some embodiments relate to the fusion protein as defined above comprising a hyaluronic acid binding protein and a TNFalpha specific protein for use in the treatment or prevention of non-infectious uveitis.

Therapeutic proteins for use in the treatment of non-infectious uveitis may be selected from the group of but not limited to Adalimumab, Infliximab, Interferon Alpha-2a, Interleukin-2, Tacrolimus, Mycophenolate Mofetil, Anakinra, and Tocilizumab.

Other therapeutic proteins for use in the treatment of eye diseases may be selected from the group of but not limited to Teprotumumab (for the treatment of thyroid eye disease), Cenegermin (for the treatment of neurotrophic keratitis), IFalpha 2a (for the treatment of ocular surface squamous neoplasia), Cyclosporine A or Lifitegrast (for the treatment of dry eye disease), recombinant PDGF-BB (for the treatment of proliferative vitreoretinophathy), Mephalan (for the treatment of retinoblastoma), Tacrolimus (for the treatment of atopic keratoconjunctivity), Ocriplasmin (for the treatment of symptomatic vitreomacular adhesion).

Some embodiments relate to methods for treating a subject with a disorder that affects the eye including administering to the eye a therapeutically effective amount of the fusion protein as defined herein.

The present invention provides a pharmaceutical composition comprising a fusion polypeptide of the invention as described herein, and a therapeutically acceptable carrier and/or diluent.

The present invention provides a fusion polypeptide of the invention as described herein, or a pharmaceutical composition comprising a fusion polypeptide of the invention as described herein, for use in medicine.

The present invention further provides a method for the prevention and/or treatment of an eye disease in a subject in need thereof, comprising administering to the subject a therapeutically effective amount of a fusion polypeptide of the invention as described herein. Typically, the fusion polypeptide is comprised by a pharmaceutical composition. Accordingly, the therapeutic method comprises the administration of a therapeutically effective amount of a pharmaceutical composition comprising a fusion polypeptide of the invention as described herein. Preferably, the subject is a human subject.

In various preferred embodiments, the eye disease is an angiogenic eye disease, more specifically a VEGF-A-associated eye disease. In various other embodiments, the eye disease is a neovascular eye disease, more specifically a VEGF-A-associated or VEGF-A-induced neovascular eye disease. Accordingly, as described herein, the eye disease preferably is a VEGF-A-associated or VEGF-A-induced eye disease. In various embodiments, the eye disease may be any of the eye disease described herein above. In preferred embodiments, the eye disease is any of age-related macular degeneration (AMD), diabetic retinopathy (DR), diabetic macular edema, retinal vein occlusion, or other.

The present invention further provides a method for the prevention and/or treatment of a joint disease in a subject in need thereof, comprising administering to the subject a therapeutically effective amount of a fusion polypeptide of the invention as described herein. Typically, the fusion polypeptide is comprised by a pharmaceutical composition. Accordingly, the therapeutic method comprises the administration of a therapeutically effective amount of a pharmaceutical composition comprising a fusion polypeptide of the invention as described herein. Preferably, the subject is a human subject.

In various embodiments of the methods for the prevention and/or treatment disclosed herein, the fusion polypeptide of the invention, or the pharmaceutical composition comprising a fusion polypeptide of the invention, is administered by topical administration or by intraocular aministration. Preferably, the intraocular administration is via intravitreal route (intravitreal administration). The topical administration or intraocular aministration, including the intravitreal route as the preferred intraocular administration, are the administration routes in the treatment or prevention of diseases of the eye, in particular eye diseases as described herein.

With regard to methods for the prevention and/or treatment of a joint disease as described herein, a preferred route of administration is inside or into a joint. As described herein, a joint is a place in the body where two bones are connected. The preferred route of administration for treating joint diseases disclosed herein is typically described as intra-articular injection. As described herein, in various embodiments of the present invention, joint diseases (or joint injuries) do not include joint fractures and/or intra-articular fractures.

Compositions. Various embodiments relate to a composition comprising the fusion protein as defined above comprising a hyaluronic acid binding protein and therapeutic protein. The composition optionally may contain further auxiliary agents and excipients known per se. These include for example but are not limited to stabilizing agents, surface-active agents, salts, buffers, coloring agents etc. The compositions can be in the form of a liquid preparation, a lyophilisate, an aerosol, in the form of powders, granules, in the form of an emulsion or a liposomal preparation. Various embodiments relate to a pharmaceutical composition for the treatment of diseases affecting the eye or joint comprising a fusion protein as disclosed herein, and a pharmaceutically acceptable carrier and/or diluent. A pharmaceutically acceptable carrier may include solvents, dispersion meida, coatings, antibacterial and antifungal agents, isotonic and absorption delaying agents, and the like that are physiologically compatible. The pharmaceutical composition comprising a fusion protein as defined above can be used for treatment of eye diseases or for the treatment of joint diseases. The pharmaceutical composition may be suitable for intravitreal administration.

The composition for the treatment of eye disorders should be fluid and sterile. In some cases, isotonic agents, polyalcohols, and sodium chloride may be included in the composition. The composition may include an agent which delays absorption, for example, gelatin or aluminum monostearate. The type of pharmaceutical preparation depends on the type of eye disease to be treated, the route of administration, the severity of the disease, the patient to be treated and other factors known to those skilled in the art of medicine.

The compositions contain a therapeutically effective dose of the fusion protein as defined above. The compositions contain a diagnostically effective dose of the hyaluron binding protein as defined above. The amount of fusion protein to be administered depends on the organism to be treated, the type of disease, the age and weight of the patient and further factors known per se. Depending on the galenic preparation these compositions can be administered by injection, or by other conventionally employed methods of application for the treatment of eye diseases. In various embodiments, the composition is suitable for delivery to the eye by intravitreal, topical ophthalmic, intraretinal, subretinal, suprachoroidal and intracameral delivery.

Various embodiments relate to a method for treating or diagnosing a subject with an eye disease, comprising administering to the eye of the subject a fusion protein or a hyaluronic acid binding protein as defined above. Preferred is a method for treating a subject with an eye disease selected from neovascular eye diseases and inflammatory eye diseases.

**Preparation of fusion proteins.** A fusion protein as defined above may be prepared by any of the many conventional and well-known techniques such as plain organic synthetic strategies, solid phase-assisted synthesis techniques, fragment ligation techniques or by commercially available automated synthesizers. On the other hand, they may also be prepared by conventional recombinant techniques alone or in combination with conventional synthetic techniques. Furthermore, they may also be prepared by cell-free *in vitro* transcription/translation or in combination with conventional synthetic techniques.

Various embodiments relate to an isolated polynucleotide encoding a fusion protein as defined above. The invention also encompasses polypeptides encoded by said polynucleotides. The invention further provides an expression vector comprising said polynucleotide, and a host cell comprising said polynucleotide or said expression vector.

Various embodiments relate to a **method for the production** of a fusion protein as defined above comprising culturing of a host cell under suitable conditions in order to obtain said fusion protein and optionally isolating said fusion protein.

Various embodiments relate to a polynucleotide encoding a fusion protein as described above. The invention further provides an expression vector comprising said polynucleotide, and a host cell comprising said isolated polynucleotide or the expression vector.

Various embodiments relate to a method for the production of a fusion protein as described above comprising culturing of a host cell under suitable conditions which allow expression of said fusion protein and optionally isolating said fusion protein.

For example, one or more polynucleotides which encode for fusion protein may be expressed in a suitable host and the produced fusion protein can be isolated. Vectors comprising said polynucleotides are covered herein. A further embodiment relates to a vector comprising said nucleic acid molecule. A vector means any molecule or entity (e.g., nucleic acid, plasmid, bacteriophage or virus) that can be used to transfer protein coding information into a host cell. Furthermore, an isolated cell is disclosed comprising said nucleic acid molecule or said vector. Suitable host cells include prokaryotes or eukaryotes. Various mammalian or insect cell culture systems can also be employed to express recombinant proteins.

An embodiment also relates to a host cell or a non-human host carrying said vector. A host cell is a cell that has been transformed with a nucleic acid sequence and thereby expresses a gene of interest.

Suitable conditions for culturing prokaryotic or eukaryotic host cells are well known to the person skilled in the art. Cultivation of cells and protein expression for the purpose of protein production can be performed at any scale, starting from small volume shaker flasks to large fermenters, applying technologies well-known to any skilled in the art.

One embodiment is directed to a method for the preparation of a binding protein as detailed above, said method comprising the following steps: (a) preparing a nucleic acid encoding a fusion protein as described above; (b) introducing said nucleic acid into an expression vector; (c) introducing said expression vector into a host cell; (d) cultivating the host cell; (e) subjecting the host cell to culturing conditions under which fusion protein is expressed, thereby producing a fusion protein as defined herein; (f) optionally isolating the fusion binding protein produced in step (e); and (g) optionally conjugating the fusion protein with further functional moieties as defined herein.

In general, isolation of purified fusion protein from the cultivation mixture can be performed applying conventional methods and technologies well known in the art, such as centrifugation, precipitation, flocculation, different embodiments of chromatography, filtration, dialysis, concentration and combinations thereof, and others. Chromatographic methods are well-known in the art and comprise without limitation ion exchange chromatography, gel filtration chromatography (size exclusion chromatography), hydrophobic interaction chromatography, or affinity chromatography.

For simplified purification, the fusion protein can be fused to other peptide sequences having an increased affinity to separation materials. Preferably, such fusions are selected that do not have a detrimental effect on the functionality of the fusion protein or can be separated after the purification due to the introduction of specific protease cleavage sites. Such methods are also known to those skilled in the art.

Further encompassed by the present invention is:
A hyaluronic acid binding protein comprising an amino acid sequence with at least 80 % or at least 85 % sequence identity to SEQ ID NO: 3 or SEQ ID NO: 4 wherein the protein has (i) at the position corresponding to position 8 in SEQ ID NO: 3 or SEQ ID NO: 4 any amino acid residue except arginine (R), (ii) at the position corresponding to position 13 in SEQ ID NO: 3 or SEQ ID NO: 4 any amino acid residue except lysine (K), and (iii) at the position corresponding to position 40 in SEQ ID NO: 3 or SEQ ID NO: 4 any amino acid except arginine (R). All embodiments described elsewhere herein in relation to the aspects of the present invention as reflected in the Summary of the Invention equally apply to this further aspect of the invention.

### EXAMPLES

The following Examples are provided for further illustration of the invention. The invention, however, is not limited thereto, and the following Examples merely show the practicability of the invention on the basis of the above description. For a complete disclosure of the invention reference is made also to the literature cited in the application which is incorporated completely into the application by reference.

### EXAMPLE 1. Mammalian expression

The N-terminal unit of the fusion proteins were comprised of the second extracellular domain of VEGFR-1 and the third extracellular domain of VEGFR-2, connected to a human IgG1-Fc part. This part is identical to Aflibercept (SEQ ID NO: 9). Aflibercept was fused at the C-terminus of the Fc to a 15 amino acid GS-linker (SEQ ID NO: 10) and the hyaluronic binding domain of SEQ ID NO: 1, 2, 3, or 4, respectively. The fusion porteins are expressed as single polypeptide chain which forms a homo-dimer during post-translational processing. The amino acid sequences of the bi-functional fusion proteins are shown in SEQ ID NOs: 5-8. A schematic drawing of a fusion protein of the invention is shown in **Figure 1****.**

Plasmids carrying the genes of the respective proteins were amplified in *E.coli* and purified with standard methods. Mammalien HEK293 cells were transfected with the plasmids and cultivated for several days to allow for protein expression.

### EXAMPLE 2. Purification

Supernatant from the cell culture was clarified and processed on a ProteinA capture column. Protein was **eluted** and further purified by anion exchange chromatography. Protein was then diafiltered into the formulation buffer and concentrated to 10 mg/ml. Quality assessment was performed by a number of methods comprising endotoxin content, aggregate content by size-exclusion chromatography, capillary iso-electric focusing, capillary gel electrophoresis and host-cell protein determination, as shown in **Table 1.** In **Table 1,** SE-UPLC refers to size-exclusion ultra performance liquid chromatography, chromogenic LAL refers to Limulus amebocyte lysate Test, iclEF refers to: imaged capillary isoelectric focusing, and IP refers to isoelectric point.

**Table 1. Purification of fusion proteins**

| **parameter** | **test method** | **unit** | **result for fusion proteins** | | | |
|---|---|---|---|---|---|---|
| | | | SEQ 8 | SEQ 7 | SEQ 5 | SEQ 6 |
| concentration | absorption at 280nm | mg/ml | 10.3 | 10.2 | 9.4 | 9.6 |
| aggregation | SE-UPLC | % | 100 | 98 | 96 | 97 |
| impurities | host cell protein ELISA | ng/mg | 73 | 37 | 37 | 31 |
| Endotoxin | Chromogenic LAL | EU/mg | <0.01 | <0.01 | <0.01 | <0.01 |
| purity | SDS-PAGE reduced | % | 95.9 | 96.1 | 96.2 | 96.2 |
| purity | SDS-PAGE nonreduced | % | 96.8 | 92.6 | 93.7 | 93.1 |
| molecular weight | SDS-PAGE nonreduced | kDa | 135 | 137 | 157 | 159 |
| IP and charge profile | icIEF | | 8.9-9.2 | 6.9-8.9 | 7.0-7.3 | 6.9-7.2 |

### EXAMPLE 3. Binding of fusion proteins to hyaluronic acid and to the target (KD-determination of binding proteins via ELISA)

Elisa microtiter plates were coated with vitreous from human or rabbit eyes, respectively, incubated overnight and washed. Compounds were added in different concentrations ranging from 50 µg/ml to 0.003 µg/ml and incubated for 2 h. Plates were washed and incubated with a secondary antibody directed against human Fc and coupled with HRP. After further washing the HRP substrate was added and the assay developed for about 20 min and the color measured in a plate reader. The OD values were plotted against the concentration and the resulting cuvers fitted with a 4-parameter logistic regression to obtain affinity values. Graphs showing the binding curves are shown **in** **FIGURE 2** and **FIGURE 3****,** EC50 values are summarized in **Table 2.**

**Table 2. Binding analysis of fusion proteins**

| | | **Affinity / EC50 in nM** | | |
|---|---|---|---|---|
| **SEQ ID NO:** | **comprising SEQ ID NO:** | **human vitreous** | **rabbit vitreous** | **VEGF** |
| 5 | 1 | 67 | 72 | <1.0 |
| 6 | 2 | 24 | 30 | <1.0 |
| 7 | 3 | 0.08 | 0.09 | <1.0 |
| 8 | 4 | 0.40 | 0.40 | <1.0 |

### EXAMPLE 4. Pharmacokinetic study in rabbits

The fusion proteins of SEQ ID NO: 5, SEQ ID NO: 6, SEQ ID NO: 7, SEQ ID NO: 8, and Aflibercept as control were analysed in 4 sub-groups (each n=12) on day 4, 12, 20, 28 after injection of 17 pmol protein to about 4-6 months old rabbits *(Oryctolagus cuniculus)* in good health and at 1.3-2.0 kg weight. Study groups were assigned according to Standard Operating Procedures. All animals were weighed prior to dosing, weekly, and at time of necropsy. A veterinary ophthalmologist performed complete ocular examinations. The half-life of fusion protein SEQ ID NO: 8 was 11.4 days, of fusion protein SEQ ID NO: 7 7.71 days, of fusion protein SEQ ID NO: 6 6.17 days, and of fusion protein SEQ ID NO: 5 4.79 days, all compared to Aflibercept (3.36 days half-life). **FIGURE 4** and **FIGURE 5** show that the hyaluronic acid binding proteins significantly prolong half-life of Aflibercept. The fusion proteins exhibit superior half-life compare to Aflibercept without inflammation or other indications of irritation to the eye in rats and rabbits. Specifically, taken at 4 days after application (administration), the fusion protein of the invention demonstrates at least 1.5 fold higher concentration (or, stated otherwise, at least 50% higher concentration) than Aflibercept.

### EXAMPLE 5. Efficacy study in rabbits

Fusion protein SEQ ID NO: 7, fusion protein SEQ ID NO: 8, and Aflibercept and vehicle buffer as control were analysed in 5 sub-groups (each n=12, except vehicle n=6) on day 4, 8, 12, 28 and 42 after injection of 17 pmol protein to about 4-6 months old rabbits *(Oryctolagus cuniculus)* in good health and at 1.3-2.0 kg weight. 20 pMol of VEGF was injected 2 days prior to the day of analysis. On the day of analysis, fluorescein was applied systemically and fluorescence angiograms taken from each eye of all rabbits. Leakage scores were assigned in a blinded fashion by experienced operators. Free VEGF induces leakage of the vessels in the retina. If sufficient amount of active compound is present to neutralize VEGF, no leakage is observed. A score of 0 corresponds to no leakage, a score of 4 to maximal leakage. Therefore, a low score over a long time is indicative of a long presence and half-life of the compound in the eye.

Study groups were assigned according to Standard Operating Procedures. All animals were weighed prior to dosing, weekly, and at time of necropsy. Results are shown in **FIGURE 6****.**

### EXAMPLE 6. Protease stability of fusion proteins

Fusion protein SEQ ID NO: 8 and fusion protein SEQ ID NO: 7, and a fusion protein not having proline in position 8 (P), no glycine (G) in position 13 and no glutamine (Q) in position 40 (referred to as control; 8R, 13K, 40R) were analysed for degradation by trypsin. In particular, the control fusion protein is a fusion corresponding to SEQ ID NO: 8 without the proline in position 8 (P), the glycine (G) in position 13 and the glutamine (Q) in position 40. The fusion proteins were incubated for 0, 2, 5, 10, 20, and 30 min in 0.0082 mg/ml trypsin (conc) and analysed by SDS-PAGE and QuickQuant (Spectroscopy). The results show that fusion proteins SEQ ID NO: 8 and SEQ ID NO: 7 were at least 1.7fold (or, stated otherwise, about 2fold) more stable to degradation by the protease trypsin within 10 minutes at 37°C in comparison to the control.

**Table 3. Protease stability of fusion proteins comprising hyaluronic binding proteins SEQ ID NO: 3 and SEQ ID NO: 4**

| **time** | **control** | **SEQ ID NO: 8 (comprising SEQ ID NO: 4)** | **SEQ ID NO: 8 x fold more stable compared to control** | **SEQ ID NO: 7 (comprising SEQ ID NO: 3)** | **SEQ ID NO: 7 x fold more stable compared to control** |
|---|---|---|---|---|---|
| **t0** | 100 | 100 | | 100 | |
| **t2** | 69 | 78 | 1.1 | 83 | 1.2 |
| **t5** | 56 | 72 | 1.2 | 62 | 1.1 |
| **t10** | 31 | 53 | 1.7 | 57 | 1.8 |

### SEQUENCE LISTING

SEQ ID NO: 1 hyaluronic acid binding protein 
VVFHYRPARGRYALNFAEAQQACLEQDAVIASPEQLQAAWEDGLDQCDAGWLSDQTVRYPIT
TPREPCGGDKDGFPGVRSYGFRDPDETYDVYCFASALEGEVFYLTPPEKLTFEEAAAACRRR
GAALAKVGQLYAAWKFGLDDRCDAGWLADGSVRYPIVTPRPRCGGPLPAGVRTVGFFPNQT
GFPDPASRFGAYCFR
SEQ ID NO: 2 hyaluronic acid binding protein 
VVFHYRPGSGRYTLNFEEAQQACLEQGAVIASPEQLQAAYEDGLDQCDAGWLSDQTVRYPIT
TPREPCYGDKDSFPGVRSYGVRDPDETYDVYCFASELEGEVFYLTPPEKLTLEEARAACRRR
GARLAKVGQLYAAWKFGLDDRCDAGWLADGSVRYPIVTPRPRCGGPLPAGVRTVYFFPNQT
GFPDPASRYGAYCFR
SEQ ID NO: 3 hyaluronic acid binding protein 
GVFHLESPSGKYGLTFDEAREACEAEGATLATYKQLEAAQKAGFHLCSAGWLATGRVAYPTA
KPSPNCGSGRVGIVDYGPRPNKSEQWDAYCYRPHA
SEQ ID NO: 4 hyaluronic acid binding protein 
GVYHREAPSGKYGLTYAEAKAVCEFEGGHLATYKQLEAAQKIGFHVCAAGWMAKGRVGYPIV
KPGPNCGFGKTGIIDYGIRLNRSERWDAYCYNPHA
SEQ ID NO: 5 Fusion protein comprising hyaluronic acid binding protein SEQ ID NO: 1 
SDTGRPFVEMYSEIPEIIHMTEGRELVIPCRVTSPNITVTLKKFPLDTLIPDGKRIIWDSRKGFIISN
ATYKEIGLLTCEATVNGHLYKTNYLTHRQTNTIIDVVLSPSHGIELSVGEKLVLNCTARTELNVGI
DFNWEYPSSKHQHKKLVNRDLKTQSGSEMKKFLSTLTIDGVTRSDQGLYTCAASSGLMTKKN
STFVRVHEKDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGGGGGSGGGGSGGGGS
VVFHYRPARGRYALNFAEAQQACLEQDAVIASPEQLQAAWEDGLDQCDAGWLSDQTVRYPIT
TPREPCGGDKDGFPGVRSYGFRDPDETYDVYCFASALEGEVFYLTPPEKLTFEEAAAACRRR
GAALAKVGQLYAAWKFGLDDRCDAGWLADGSVRYPIVTPRPRCGGPLPAGVRTVGFFPNQT
GFPDPASRFGAYCFR
 
SEQ ID NO: 6 Fusion protein comprising hyaluronic acid binding protein SEQ ID NO: 2
SDTGRPFVEMYSEIPEIIHMTEGRELVIPCRVTSPNITVTLKKFPLDTLIPDGKRIIWDSRKGFIISN
ATYKEIGLLTCEATVNGHLYKTNYLTHRQTNTIIDVVLSPSHGIELSVGEKLVLNCTARTELNVGI
DFNWEYPSSKHQHKKLVNRDLKTQSGSEMKKFLSTLTIDGVTRSDQGLYTCAASSGLMTKKN
STFVRVHEKDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGGGGGSGGGGSGGGGS
VVFHYRPGSGRYTLNFEEAQQACLEQGAVIASPEQLQAAYEDGLDQCDAGWLSDQTVRYPIT
TPREPCYGDKDSFPGVRSYGVRDPDETYDVYCFASELEGEVFYLTPPEKLTLEEARAACRRR
GARLAKVGQLYAAWKFGLDDRCDAGWLADGSVRYPIVTPRPRCGGPLPAGVRTVYFFPNQT
GFPDPASRYGAYCFR
SEQ ID NO: 7 Fusion protein comprising hyaluronic acid binding protein SEQ ID NO: 3 
SDTGRPFVEMYSEIPEIIHMTEGRELVIPCRVTSPNITVTLKKFPLDTLIPDGKRIIWDSRKGFIISN
ATYKEIGLLTCEATVNGHLYKTNYLTHRQTNTIIDVVLSPSHGIELSVGEKLVLNCTARTELNVGI
DFNWEYPSSKHQHKKLVNRDLKTQSGSEMKKFLSTLTIDGVTRSDQGLYTCAASSGLMTKKN
STFVRVHEKDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGGGGGSGGGGSGGGGS
GVFHLESPSGKYGLTFDEAREACEAEGATLATYKQLEAAQKAGFHLCSAGWLATGRVAYPTA
KPSPNCGSGRVGIVDYGPRPNKSEQWDAYCYRPHA
SEQ ID NO: 8 Fusion protein comprising hyaluronic acid binding protein SEQ ID NO: 4 
SDTGRPFVEMYSEIPEIIHMTEGRELVIPCRVTSPNITVTLKKFPLDTLIPDGKRIIWDSRKGFIISN
ATYKEIGLLTCEATVNGHLYKTNYLTHRQTNTIIDVVLSPSHGIELSVGEKLVLNCTARTELNVGI
DFNWEYPSSKHQHKKLVNRDLKTQSGSEMKKFLSTLTIDGVTRSDQGLYTCAASSGLMTKKN
STFVRVHEKDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGGGGGSGGGGSGGGGS
GVYHREAPSGKYGLTYAEAKAVCEFEGGHLATYKQLEAAQKIGFHVCAAGWMAKGRVGYPIV
KPGPNCGFGKTGIIDYGIRLNRSERWDAYCYNPHA
SEQ ID NO: 9 Aflibercept 
SDTGRPFVEMYSEIPEIIHMTEGRELVIPCRVTSPNITVTLKKFPLDTLIPDGKRIIWDSRKGFIISN
ATYKEIGLLTCEATVNGHLYKTNYLTHRQTNTIIDVVLSPSHGIELSVGEKLVLNCTARTELNVGI
DFNWEYPSSKHQHKKLVNRDLKTQSGSEMKKFLSTLTIDGVTRSDQGLYTCAASSGLMTKKN
STFVRVHEKDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPG
SEQ ID NO: 10 Linker
   GGGGSGGGGSGGGGS

### Aspects and embodiments of the invention include:

1. A hyaluronic acid binding protein comprising an amino acid sequence with at least 80 % sequence identity to SEQ ID NO: 3 or SEQ ID NO: 4 wherein the protein has (i) a proline (P) at the position corresponding to position 8 in SEQ ID NO: 3 or SEQ ID NO: 4, (ii) a glycine (G) at the position corresponding to position 13 in SEQ ID NO: 3 or SEQ ID NO: 4, and (iii) a glutamine (Q) at the position corresponding to position 40 in SEQ ID NO: 3 or SEQ ID NO: 4, and wherein the hyaluronic acid binding protein has improved stability against proteolytic degradation as compared to the same protein not comprising the amino acid residues under the above (i), (ii), and (iii).
2. The hyaluronic acid binding protein of item 1, wherein the protein has a specific binding affinity for hyaluronic acid of at least 100 nM.
3. A fusion protein comprising
   a) a hyaluronic acid binding protein according to item 1 or item 2, and
   b) a therapeutic protein, preferably for the treatment of eye diseases or for the treatment of diseases of the joints.
4. The fusion protein of item 3, wherein the therapeutic protein is a monoclonal antibody, fusion protein, recombinant protein, peptide drug, growth factor, immunosuppressant, enzyme, or protein inhibitor.
5. The fusion protein of item 3 or 4, comprising an amino acid sequence with at least 80 % sequence identity to SEQ ID NO: 7 or SEQ ID NO: 8.
6. The fusion protein of item 3 or 4, wherein the therapeutic protein for the treatment of eye diseases is selected from Aflibercept, Ranibizumab, Bevacizumab, Brolucizumab, Farcimab, Adalimumab, Infliximab, Teprotumumab, Cenegermin, Interferon alpha-2a, Cyclosporine A, Lifitegrast, recombinant human platelet-derived growth factor (PDGF-BB), Melphalan, Tacrolimus, Mycophenolate Mefetil, Ocriplasmin, Anakinra, Toclizumab, Pegcetacoplan, Avacincaptad peptol, or derivatives of any of those, or biosimilars.
7. The fusion protein of item 3 or 4, wherein the fusion protein comprises two subunits one that binds to hyaluronic acid and one that binds to a disease target, respectively, wherein the first subunit comprises a hyaluronic acid binding protein according to item 1 or 2, and wherein the second subunit is a therapeutic protein.
8. The fusion protein of item 3 or 4 for use in the treatment of eye diseases.
9. The fusion protein of item 8, wherein the fusion protein has at least 1.5 fold higher concentration in the vitreous body 4 days after application as compared to the concentration of the therapeutic protein under the same conditions.
10. The fusion protein of item 8, wherein the fusion protein has at least 30% longer half-life compared to the therapeutic protein, in particular wherein the half-life means vitreal half-life.
11. A composition comprising the hyaluronic acid binding protein of item 1 or 2, or the fusion protein of item 3 or 4, for delivery to the eye or the joints.
12. A composition comprising the fusion protein according of item 3 or 4 and a therapeutically acceptable carrier and/or diluent for the treatment of eye diseases or diseases of the joints.
13. A composition comprising the hyaluronic acid binding protein of item 1 or item 2 and a diagnostically acceptable carrier and/or diluent for the diagnosis of eye diseases or diseases of the joints.
14. A method for treating an eye disease, the method comprising administering to the eye of a subject in need thereof a therapeutically effective amount of the fusion protein of item 3 or 4, or the composition of item 11, in an amount and via a route sufficient to treat the eye disease.
15. A method for producing a hyaluronic acid binding protein according to item 1 or 2, or a fusion protein of item 3 or 4, comprising the steps of a) culturing of a host cell comprising a polynucleotide encoding the hyaluronic acid binding protein according to item 1 or 2, or the fusion protein of item 3 or 4, under conditions suitable to obtain said protein and b) isolating said protein produced from the host cell or from a medium in which the host cell was cultured.

## Claims

1. A hyaluronic acid binding protein comprising an amino acid sequence with at least 80 % sequence identity to SEQ ID NO: 3 or SEQ ID NO: 4 wherein the protein has (i) a proline (P) at the position corresponding to position 8 in SEQ ID NO: 3 or SEQ ID NO: 4, (ii) a glycine (G) at the position corresponding to position 13 in SEQ ID NO: 3 or SEQ ID NO: 4, and (iii) a glutamine (Q) at the position corresponding to position 40 in SEQ ID NO: 3 or SEQ ID NO: 4, and wherein the hyaluronic acid binding protein has improved stability against proteolytic degradation as compared to the same protein not comprising the amino acid residues under the above (i), (ii), and (iii).

2. The hyaluronic acid binding protein of claim 1, wherein the protein has a specific binding affinity for hyaluronic acid of at least 100 nM.

3. A fusion protein comprising
a) a hyaluronic acid binding protein according to claim 1 or claim 2, and
b) a therapeutic protein, preferably for the treatment of eye diseases or for the treatment of diseases of the joints.

4. The fusion protein of claim 3, wherein the therapeutic protein is a monoclonal antibody, fusion protein, recombinant protein, peptide drug, growth factor, immunosuppressant, enzyme, or protein inhibitor.

5. The fusion protein of claim 3 or 4, comprising an amino acid sequence with at least 80 % sequence identity to SEQ ID NO: 7 or SEQ ID NO: 8.

6. The fusion protein of claim 3 or 4, wherein the therapeutic protein for the treatment of eye diseases is selected from Aflibercept, Ranibizumab, Bevacizumab, Brolucizumab, Farcimab, Adalimumab, Infliximab, Teprotumumab, Cenegermin, Interferon alpha-2a, Cyclosporine A, Lifitegrast, recombinant human platelet-derived growth factor (PDGF-BB), Melphalan, Tacrolimus, Mycophenolate Mefetil, Ocriplasmin, Anakinra, Toclizumab, Pegcetacoplan, Avacincaptad peptol, or derivatives of any of those, or biosimilars.

7. The fusion protein of claim 3 or 4, wherein the fusion protein comprises two subunits one that binds to hyaluronic acid and one that binds to a disease target, respectively, wherein the first subunit comprises a hyaluronic acid binding protein according to claim 1 or 2, and wherein the second subunit is a therapeutic protein.

8. The fusion protein of claim 3 or 4 for use in the treatment of eye diseases.

9. The fusion protein of claim 8, wherein the fusion protein has at least 1.5 fold higher concentration in the vitreous body 4 days after application as compared to the concentration of the therapeutic protein under the same conditions.

10. The fusion protein of claim 8, wherein the fusion protein has at least 30% longer half-life compared to the therapeutic protein, in particular wherein the half-life means vitreal half-life.

11. A composition comprising the hyaluronic acid binding protein of claim 1 or 2, or the fusion protein of claim 3 or 4, for delivery to the eye or the joints.

12. A composition comprising the fusion protein according of claim 3 or 4 and a therapeutically acceptable carrier and/or diluent for the treatment of eye diseases or diseases of the joints.

13. A composition comprising the hyaluronic acid binding protein of claim 1 or claim 2 and a diagnostically acceptable carrier and/or diluent for the diagnosis of eye diseases or diseases of the joints.

14. A method for treating an eye disease, the method comprising administering to the eye of a subject in need thereof a therapeutically effective amount of the fusion protein of claim 3 or 4, or the composition of claim 11, in an amount and via a route sufficient to treat the eye disease.

15. A method for producing a hyaluronic acid binding protein according to claim 1 or 2, or a fusion protein of claim 3 or 4, comprising the steps of a) culturing of a host cell comprising a polynucleotide encoding the hyaluronic acid binding protein according to claim 1 or 2, or the fusion protein of claim 3 or 4, under conditions suitable to obtain said protein and b) isolating said protein produced from the host cell or from a medium in which the host cell was cultured.
